(19) Europäisches Patentamt
European Patent Office
Office européen des brevets

(11) EP 1 856 267 B1

(12) **EUROPEAN PATENT SPECIFICATION**

(45) Date of publication and mention of the grant of the patent:
**26.08.2009 Bulletin 2009/35**

(21) Application number: **06710018.0**

(22) Date of filing: **03.03.2006**

(51) Int Cl.:
***C12N 15/85*** *(2006.01)*

(86) International application number:
**PCT/GB2006/000802**

(87) International publication number:
**WO 2006/095156 (14.09.2006 Gazette 2006/37)**

(54) **VECTORS COMPRISING NOVEL REGULATORY ELEMENTS**

NEUE REGULATORISCHE ELEMENTE ENTHALTENDE VEKTOREN

VECTEURS COMPRENANT DES NOUVEAUX ELEMENTS REGULATEURS

(84) Designated Contracting States:
**AT BE BG CH CY CZ DE DK EE ES FI FR GB GR HU IE IS IT LI LT LU LV MC NL PL PT RO SE SI SK TR**

(30) Priority: **05.03.2005 GB 0504587**
**14.03.2005 US 661553 P**

(43) Date of publication of application:
**21.11.2007 Bulletin 2007/47**

(73) Proprietor: **MILLIPORE CORPORATION**
**Billerica**
**Massachusetts 01821 (US)**

(72) Inventors:
- **WILLIAMS, Steven, Geraint**
  **Hough, Near Crewe, Cheshire CW2 5RE (GB)**
- **GAWN, Jonathan**
  **Crewe, Cheshire CW2 5HZ (GB)**
- **IRVINE, Alistair, Simpson**
  **8915 Hausen am Albis (CH)**

(74) Representative: **Ward, Siobhan et al**
**Harrison Goddard Foote**
**40-43 Chancery Lane**
**London WC2A 1JA (GB)**

(56) References cited:
**WO-A-02/081677** **WO-A-02/099089**

- **YIN C-Y ET AL: "GUINEA-PIG CYTOMEGALOVIRUS IMMEDIATE EARLY TRANSCRIPTION" JOURNAL OF VIROLOGY, vol. 64, no. 4, 1990, pages 1537-1548, XP002384586 ISSN: 0022-538X**
- **ISOM H C ET AL: "CHARACTERIZATION OF GUINEA-PIG CYTOMEGALOVIRUS DNA" JOURNAL OF VIROLOGY, vol. 49, no. 2, 1984, pages 426-436, XP002384587 ISSN: 0022-538X**
- **GAO M ET AL: "CHARACTERIZATION OF THE GUINEA-PIG CYTOMEGALOVIRUS GENOME BY MOLECULAR CLONING AND PHYSICAL MAPPING" JOURNAL OF VIROLOGY, vol. 52, no. 2, 1984, pages 436-447, XP002384588 ISSN: 0022-538X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 856 267 B1

Printed by Jouve, 75001 PARIS (FR)

## Description

## BACKGROUND

The invention relates to the field of recombinant DNA technology and, in particular, the development of vectors for the expression of recombinant proteins. Expression of heterologous genes in eukaryotic cells is a fundamental aspect of biotechnology with many academic and commercial applications. Expression of such genes requires transcription by RNA polymerase II (Pol II), which is driven by cis-acting genetic elements known as promoters and enhancers.

In simple terms, promoters are directional elements that act to initiate transcription of sequences situated less than 100 (and usually less than 50) nucleotide base pairs (bp) downstream. They contain a number of short consensus nucleotide sequences that act as binding sites for various proteins that participate in the initiation of transcription and the assembly of a multi-subunit complex known as the pre-initiation complex (McKnight and Tjian, 1987, Cell 46: 795-805). In most genes, this occurs at a very widely conserved sequence known as the TATA box (TATAAA) to which the TATA box-binding protein (TBP, a subunit of the general transcription factor TFIID) binds. There follows an ordered assembly of more than ten further transcription factors to finally form the Pol II holoenzyme complex. RNA transcription actually starts at an initiator site about 25-30 bases downstream (Breathnach and Chambon, 1981, Annu Rev Biochem 50: 349-393) to which TBP also binds.

Most functional promoters contain further upstream promoter elements (UPEs), of which the most highly conserved are the CAAT box (CCAAT, the binding site for the transcription factors CBF, C/EBP and NF-1), about 70-200bp upstream, and the GC box (GGGCGG, binding site for the general transcription factor Sp-1) a similar distance upstream. Although basal levels of transcription occur from the TATA box alone, for most promoters at least the CAAT and GC boxes are required for optimal levels of transcription.

Enhancers are sequences that act non-directionally to increase transcription from promoters situated locally but not necessarily immediately adjacent (up to several kilobases away (Kadonaga (2004) Cell 116: 247-257). Enhancers contain short (8-12bp) consensus sequences representing the binding sites for a wide range of transcriptional activator proteins (Ondek et al, 1988, Science 236: 1237-1244) including some, such as NF-1 and SP-1 that are also associated with promoter elements. These sequences are often duplicated in tandem or inverted repeats.

In some natural transcription units, including the very active immediate/early gene transcription units of many DNA viruses such as cytomegalovirus, enhancer and promoter elements may be functionally combined into what is effectively one extended upstream element.

Promoters may be regulated, being responsive to cell type, temperature, metal ions or other factors; or constitutive, giving transcription that is unresponsive to such factors.

For many purposes a strong, constitutive promoter giving consistent, high, levels of transcription in many, if not all, cell types is highly advantageous. For many years the enhancer/promoter element driving immediate/early gene expression in human cytomegalovirus has been very widely used for driving such expression of heterologous genes in eukaryotic expression vectors (Foecking & Hoffstetter, 1986, Gene 45: 101-105).

Human cytomegalovirus (CMV) is a member of the betaherpesvirus family and is responsible for gastrointestinal and respiratory infections, hepatitis, and retinitis. As with other herpesviruses, CMV can persist in latent infections and can be reactivated in immunocompromised individuals. In cell culture, human CMV replicates productively in terminally differentiated cells such as fibroblast, epithelial, and endothelial cells and in monocyte-derived macrophages (Isomura and Stinski, 2003, J Virol 77: 3602-3614 and references therein).

During productive infection, there is an ordered expression of sets of CMV genes, designated immediate-early (IE), early, or late. The human CMV IE genes are thought to play a critical role in the efficiency of replication (reviewed in Castillo and Kowalik, 2002, Gene 290:19-34).

The region upstream of the human CMV IE promoter is divided into three regions, the modulator, the unique region, and the enhancer. The enhancer is also divided into a distal and a proximal enhancer. The distal enhancer is necessary for efficient IE gene expression and viral replication at a low MOI. Human CMVs have very strong enhancers for the expression of IE genes. The human CMV enhancer has four 18-bp repeat elements containing an NF-kB or rel binding site, five 19-bp repeat elements containing a CREB or ATF binding site, two AP-1 binding sites, and multiple SP-1 sites (Thomsen et al, 1984, Proc Natl Acad Sci USA 81: 659-663; Meier and Stinski, 1996, Intervirology 39: 331-342). The murine CMV enhancer contains six NF-□B or *rel* binding sites, one CREB or ATF binding site, and at least seven AP-1 binding sites (Dorsch-Hasler et al, 1985, Proc Natl Acad Sci USA 82: 8325-8329). The different *cis*-acting elements act individually and synergistically to stabilize the RNA polymerase II transcription initiation complex on the promoter.

A number of cytomegaloviruses predominantly infecting other host species are known, although, in many cases, the exact taxonomy and degree of cross-species relatedness is provisional. Cytomegalovirus-like viruses infecting a number of primate species (including African green monkey, Rhesus monkey and bonobo) and rodents including mouse, rat and guinea pig are recognised. Of these, only the murine and rat promoter-enhancers have been subject to detailed functional analysis. Comparison of these species with human CMV shows that the functions of the IE promoter-enhancers are not directly comparable, probably because of the presence of unrecognised *cis*-acting elements contributing to downstream transcription in cells of different species (Isomura and Stinski, 2003, J Virol 77: 3602-3614).

However, both human and murine CMV IE promoter-enhancers produce high levels of constitutive expression of heterologous genes in eukaryotic expression vectors and are widely used in biotechnology. Such use of the human CMV promoter was disclosed in US 5,168, 062 (Stinski / University of Iowa). Use of the promoter, enhancer and functionally complete 5' (upstream) untranslated region including the first intron of the human cytomegalovirus major immediate-early gene, wherein this is not directly linked to its natural DNA coding sequence is claimed by US 5,591,639 (Bebbington /Celltech). Use of the murine CMV IE enhancer is disclosed by US 4,968,615 (Koszinowski et al)

Guinea pig CMV (GPCMV) produces a disease of guinea pigs with many similarities to the pathology of human CMV infections. Attempts to characterise the genome (Isom et al, 1984, J Virology 49: 426-436; Gao and Isom, 1984, J Virology 52: 436-447) suggested that the structural organisation of the genome was unique amongst herpesviruses. Although of a similar size to human and murine CMV, the GPCMV genome was far simpler than that of human CMV and most closely resembled that of murine CMV. However, the GPCMV genome had several unusual features, particularly in the structure of the terminal regions. Later studies of IE gene expression identified an IE region by sequence comparison with human CMV (Yin et al, 1990, J Virol 64: 1537-1548) and the expression and processing of IE transcripts was analysed. However, there was no analysis of the usefulness of the IE promoter-enhancer for the expression of heterologous genes.

The sequence of the 'HRv' (*Hin*d III-*Eco*RV) immediate-earfy upstream fragment of the GPCMV genome, containing the 5' end of IE1 coding sequence and the upstream promoter/enhancer regions was sequenced (Yin, 1991, Guinea pig cytomegalovirus immediate-early gene expression, PhD thesis, Pennsylvania State University, USA) and shown to contain a region of repetitive sequences, typical of a CMV IE regulatory region. Three short repeats, GP-1, GP-2 and GP-3 were identified. GP-1 is an 18-bp repeat occurring 9 times (73-100% similarity to a GGCCCGGGACTTTCCA consensus) containing an NF-KB binding site and corresponding to the HCMV 18-bp repeat. GP-2 is a 17-bp repeat occurring 10 times (86-100% similarity to a TGTCCTTTTTGGCAAA consensus) and containing a core sequence similar to the consensus SRE (serum response element). GP-3 is repeated 4 times in the proximal upstream region and contains GTGACTTTT, a sequence identified as a binding site for *c-jun* or GCN4 (Hill et al, 1984, Science 234: 451-457).

Although this work suggested that the GPCMV IE upstream region contained a strong promoter, due to the way the reporter constructs were made certain artefacts could not be excluded. Firstly, the HRv fragment also appears to include the first exon and part of the first intron of the IE1 gene. This intron contains three copies of a putative NF-1 binding site, which may have artificially boosted the apparent strength of the promoter. Secondly, the reporter constructs used to test the GPCMV fragments contained an SV40 promoter (itself a strong viral promoter), so that reporter expression resulted from the effect of a double GPCMV / SV40 promoter. As a result it is not possible to make comparisons of the GPCMV enhancer/promoter alone with other strong promoters generally, or even with other CMV IE enhancer/promoters.

The applicant's co-pending patent application PCT/GB99/02357 (WO 00/05393), incorporated by reference herein, describes elements that are responsible, in their natural chromosomal context, for establishing an open chromatin structure across a locus that consists exclusively of ubiquitously expressed, housekeeping genes. These elements are not derived from a Locus Control Region (LCR) and comprise extended methylation-free CpG islands. The term Ubiquitous Chromatin Opening Element (UCOE) has been used to describe such elements.

In mammalian DNA, the dinucleotide CpG is recognised by a DNA methyltransferase enzyme that methylates cytosine to 5-methylcytosine. However, 5-methylcytosine is unstable and is converted to thymine. As a result, CpG dinucleotides occur far less frequently than one would expect by chance. Some sections of genomic DNA nevertheless do have a frequency of CpG that is closer to that expected, and these sequences are known as "CpG islands". As used herein a "CpG island" is defined as a sequence of DNA, of at least 200bp, that has a GC content of at least 50% and an observed / expected CpG content ratio of at least 0.6 (i.e. a CpG dinucleotide content of at least 60% of that which would be expected by chance) (Gardiner-Green M and Frommer M. J Mol Biol 196, 261-282 (1987); Rice P, Longden I and Bleasby A Trends Genet 16, 276-277 (2000).

Methylation-free CpG islands are well-known in the art (Bird et al (1985) Cell 40: 91-99, Tazi and Bird (1990) Cell 60: 909-920) and may be defined as CpG islands where a substantial proportion of the cytosine residues are not methylated and which usually extend over the 5' ends of two closely spaced (0.1-3 kb) divergently transcribed genes. These regions of DNA are reported to remain hypomethylated in all tissues throughout development (Wise and Pravtcheva (1999) Genomics 60: 258-271). They are often associated with the 5' ends of ubiquitously expressed genes, as well as an estimated 40% of genes showing a tissue-restricted expression profile (Antequera, F. & Bird, A. Proc. Natl. Acad. Sci. USA 90, 1195-11999 (1993); Cross, S.H. & Bird, A.P. Curr. Opin, Genet. Dev. 5, 309-314 (1995) and are known to be localised regions of active chromatin (Tazi, J. & Bird, A. Cell 60, 909-920 (1990).

An 'extended' methylation-free CpG island is a methylation-free CpG island that extends across a region encompassing more than one transcriptional start site and/or extends for more than 300bp and preferably more than 500bp. The borders of the extended methylation-free CpG island are functionally defined through the use of PCR over the region in combination with restriction endonuclease enzymes whose ability to digest (cut) DNA at their recognition sequence is sensitive to the methylation status of any CpG residues that are present. One such enzyme is *Hpa*II, which recognises and digests at the site CCGG, which is commonly found within CpG islands, but only if the central CG residues are *not* methylated. Therefore, PCR conducted with *Hpa*II-digested DNA and over a region harbouring *Hpa*II sites, does not give an amplification product due to *Hpa*II digestion if the DNA is *unmethylated.* The PCR will *only* give an amplified product if the DNA is *methylated*. Therefore, beyond the methylation-free region *Hpa*II will not digest the DNA a PCR amplified product will be observed thereby defining the boundaries of the "extended methylation-free CpG island".

International application WO 00/05393 demonstrates that regions spanning methylation-free CpG islands encompassing dual, divergently transcribed promoters from the human TATA binding protein (*TBP*)/proteosome component-B1 (*PSMBI*) and heterogeneous nuclear ribonucleoprotein A2/B1 (*hnRNPA2*)/heterochromatin protein 1Hsγ (*HP1* $^{Hs\gamma}$) gene loci impart enhanced levels of gene expression to operably linked genes. Methylation-free CpG islands associated with actively transcribing promoters possess the ability to remodel chromatin and are thus thought to be a prime determinant in establishing and maintaining an open domain at housekeeping gene loci.

UCOEs confer an increased proportion of productive gene integration events with improvements in the level and stability of transgene expression. This has important research and biotechnological applications including the generation of transgenic animals and recombinant protein products in cultured cells.

WO 00/05393 discloses functional UCOE fragments of approximately 4.0kb, in particular, the'5.5 RNP' fragment defined by nucleotides 4102 to 8286 of Figure 21 (as disclosed on p11, lines 6 and 7). The same application discloses a '1.5kb RNP' fragment (Figs 22 and 29, derivation described on p51, lines 1 to 5). However, this fragment is actually a 2165bp *Bam*HI-*Tth*111I fragment of the '5.5 RNP' fragment described above, consisting of nucleotides 4102 to 6267 of Figure 21 of that application.

A further application , WO 02/24930, discloses artificially-constructed UCOEs composed of fragments of naturally-occurring CpG islands. A third application, WO 04/067701 describes polynucleotides comprising small functional fragments of UCOEs. Such polynucleotides comprise methylation-free CpG islands of no more than approximately 2kb, or fragments of larger such islands, of not more than approximately 2kb.

Given the importance of recombinant protein expression in biotechnology, there remains a need for improved expression vectors comprising novel promoter / enhancer combinations.

## SUMMARY OF THE INVENTION

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

As used herein, the term "operably linked" refers to a relationship of operability between elements in the polynucleotides of the invention. "Operably linked" is a term, well known to those of skill in the art, that describes a functional relationship between *cis*-acting DNA sequences. The exact structural relationship may or may not be relevant and differs for different types of elements. For a promoter, it implies an essentially adjacent (usually within less than 100bp) position 5' to the open reading frame that it drives. In the case of extended methylation-free CpG islands, it appears that a regional effect on chromatin structure is responsible for increasing the level and consistency of gene expression. By way of example, the element comprising an extended methylation-free CpG-island may be positioned 5' of the enhancer / promoter controlling transcription of the expressible gene. However, "operably-linked" embraces the possibility of its being positioned elsewhere, as long as a clear functional effect can be demonstrated.

By 'functional homologue' is meant a polynucleotide sequence capable of hybridising, under stringent conditions, to the disclosed sequence, and which has similar properties of conferring increased expression of operably-linked expressible open reading frames in two or more tissues. Stringent hybridisation/washing conditions are well known in the art. For example, nucleic acid hybrids that are stable after washing in 0.1 x SSC ,0.1% SDS at 60°C. It is well known in the art that optimal hybridisation conditions can be calculated if the sequence of the nucleic acid is known. For example, hybridisation conditions can be determined by the GC content of the nucleic acid subject to hybridisation. See Sambrook et al (1989), Molecular Cloning; A Laboratory Approach. A common formula for calculating the stringency conditions required to achieve hybridisation between nucleic acid molecules of a specified homology is:

$$T_m = 81.5^0 C + 16.6 \text{ Log } [Na^+] + 0.41[\ \%\ G + C] -0.63\ (\%\text{formamide})$$

An object of the invention is to provide novel DNA molecules and vectors containing transcriptional enhancers providing very high levels of expression of operably-linked expressible nucleic acid sequences in eukaryotic cells. Advantageously the enhancers may be used in combination with their naturally-associated promoters and / or other genetic elements that increase transcription.

The invention relates to the guinea pig cytomegalovirus early-immediate promoter / enhancer and its use in expression vectors, particularly for obtaining high levels of expression of recombinant proteins. The invention provides eukaryotic expression vectors that are capable of providing increased levels of expression in many cell types over that obtainable from human or murine CMV IE enhancer /promoter elements

The guinea pig cytomegalovirus early-immediate upstream regulatory region consists of the approximately 1500bp upstream of the IE1 gene and more especially the sequence disclosed by Figure 1 and SEQ ID NO:1. It comprises both promoter and enhancer elements. By 'promoter' is meant at least the transcriptional start site, TATA box and CAAT box, being a fragment comprising nucleotides 779 to 880 of Figure 1 (SEQ ID NO: 1) or a functional homologue thereof.

Accordingly, the invention provides an isolated polynucleotide comprising at least 100, preferably 200, and more preferably at least 500, contiguous polynucleotides of the guinea pig CMV immediate/early regulatory region as depicted in Figure 1 and SEQ ID NO:1 and an expressible polynucleotide sequence, transcription of said expressible polynucleotide sequence being driven by a promoter situated between enhancer and gene or other expressible sequence, which may be the endogenous guinea pig CMV immediate/early promoter or some other, heterologous, promoter not naturally associated with the enhancer. The expressible polynucleotide sequence is not a guinea pig CMV immediate /early gene and is not naturally operably linked with the promoter. It will be understood, by one of skill in the art, that in the case of a circular isolated polynucleotide (as in a plasmid vector) by 'between' is meant upstream of the directly operably linked expressible polynucleotide sequence (5' with respect to the sense strand), and downstream (3') of the operably linked enhancer. It is understood that such an isolated polynucleotide may comprise other promoters, not associated with expression of the inserted expressible sequence of interest (such as those required for expression of selectable markers or those associated with other elements).

Hence the isolated polynucleotide comprises

a) an element comprising at least 200, and preferably at least 500, contiguous nucleotides of SEQ ID NO:1 wherein said 200 contiguous nucleotides (or 500 mt) comprise a promoter and/or an enhancer element and
b) an element comprising an expressible polynucleotide sequence;

characterised in that said isolated polynucleotide comprises, in a 5' to 3' direction with respect to the sense strand of the expressible polynucleotide sequence, an enhancer, a single promoter, and said expressible polynucleotide sequence, and wherein said enhancer is operably linked to said promoter, which is directly operably linked to said expressible polynucleotide sequence and wherein said promoter is not naturally operably linked to said expressible polynucleotide sequence..

Preferably the isolated polynucleotide contains a 5' fragment of the immediate/early regulatory region comprising nucleotides 50 to 550 or, alternatively, a 3' fragment comprising nucleotides 275 to 775. Such fragments contain functional enhancer fragments, without the endogenous promoter.

In one embodiment, therefore, the isolated polynucleotide of the invention comprises at least the promoter from the immediate/early regulatory region of guinea pig CMV directly operably linked to an expressible nucleic acid sequence to which it is not naturally operably linked, said promoter preferably comprising nucleotides 779 to 880 of SEQ ID NO: 1. By 'directly operably linked' is meant that transcription of the gene or other expressible nucleic acid is driven directly from the promoter.

Preferably, said isolated polynucleotide further comprises the enhancer from the major immediate/early regulatory region of guinea pig CMV, more preferably comprising nucleotides 1 to 887 of SEQ ID NO:1

In one preferred embodiment, said isolated polynucleotide further comprises an extended, methylation-free CpG island operably linked to said expressible nucleic acid sequence. More preferably, said extended, methylation-free CpG island comprises one or more further promoters, particularly dual or bi-directional promoters that transcribe divergently. Hence the invention provides an isolated polynucleotide comprising at least 200 contiguous nucleotides of Figure 1 (SEQ ID NO:1), operably linked to an expressible polynucleotide sequence, and further comprising an extended, methylation-free CpG island operably linked to said expressible polynucleotide sequence. Such an extended, methylation-free CpG island may be conveniently situated adjacent to, and upstream of, the enhancer sequence. Preferably such an isolated polynucleotide comprises at least 500 contiguous polynucleotides of Figure 1 (SEQ ID NO:1), more preferably a 5' fragment of the immediate/early regulatory region comprising nucleotides 50 to 550 or, alternatively, a 3' fragment comprising nucleotides 275 to 775. Most more preferably it comprises nucleotides 1 to 887 of SEQ ID NO:1

In one embodiment, said extended, methylation-free CpG island comprises a 44kb DNA fragment spanning the human TATA binding protein gene and 12kb each of the 5' and 3' flanking sequence, or functional fragment thereof. Preferably, the functional fragment comprises a 25kb DNA fragment spanning the human TATA binding protein gene with 1 kb 5' and 5kb 3' flanking sequence or a functional fragment thereof. More preferably, the functional fragment of the TATA binding protein gene-associated extended, methylation-free CpG island is of not more than 2kb, further preferably of no more than approximately 1 kb, most preferably comprising a 987bp *Bsp*E1- *Esp*31 restriction fragment.

In a second embodiment, said extended, methylation-free CpG island comprises a 60 kb DNA fragment spanning the human hnRNP A2 gene with 30 kb 5' and 20 kb 3' flanking sequence, or a functional fragment thereof. Preferably, said functional fragment comprises a 16kb DNA fragment spanning the human hnRNP A2 gene with 5kb 5' and 1.5kb 3' flanking sequence, more preferably a fragment of the human hnRNP A2 gene of no more than 2kb, more preferably no more than 1.6kb, comprising a 1546bp Esp31 restriction fragment. Preferably, said fragment is orientated in forward orientation.

In a third embodiment, the isolated polynucleotide of the present invention comprises a fragment of the β-actin CpG island/ promoter region, preferably of human origin, more preferably a DNA fragment within the range of 100bp to 2kb spanning the human β-actin CpG island/ promoter region.

In a fourth embodiment, the isolated polynucleotide of the present invention comprises a fragment of the PDCD2 CpG island/ promoter region, preferably of human origin, more preferably a DNA fragment within the range from 100bp to 2 kb spanning the human PDCD2 CpG island/ promoter region.

In a final alternative embodiment, said extended, CpG-rich unmethylated CpG island is an artificial sequence, not occurring in nature, comprising a DNA fragment within the range from 100bp to 1.9kb spanning the human β-actin CpG island/ promoter region and a DNA fragment within the range from 100bp to 2kb spanning the human PDCD2 CpG island/ promoter region. Preferably said fragments are directly adjacent with their promoters oriented divergently.

In a further aspect the invention provides a vector comprising the isolated polynucleotide as described above. The vector may be any vector capable of transferring DNA to a cell. Preferably the vector is a eukaryotic expression vector. Such vectors comprise elements such as promoters and enhancers capable of directing and enhancing transcription in eukaryotic cells. They also preferably contain other features to facilitate and optimise their function. Such features include origins of replication selected to allow replication in the appropriate eukaryotic host cell and also in prokaryotic cells used to manufacture the vectors themselves, one or more selectable markers (often conferring resistance to antibiotics or toxins) allow selection of cells containing the vector in either cell type, elements allowing amplification of the vector or integrated fragments of it, and polylinkers or multicloning sites conveniently situated downstream of the main enhanced promoter to allow easy insertion of an expressible polynucleotide sequence (commonly referred to as an 'insert') encoding a desired polypeptide product. Such refinements are well-known in the art.

Preferably, the vector is an integrating vector or an episomal vector.

Preferred integrating vectors include recombinant retroviral vectors. A recombinant retroviral vector will include DNA of at least a portion of a retroviral genome which portion is capable of infecting the target cells. The term "infection" is used to mean the process by which a virus transfers genetic material to its host or target cell. Preferably, the retrovirus used in the construction of a vector of the invention is also rendered replication-defective to remove the effect of viral replication of the target cells. In such cases, the replication-defective viral genome can be packaged by a helper virus in accordance with conventional techniques. Generally, any retrovirus meeting the above criteria of infectiousness and capability of functional gene transfer can be employed in the practice of the invention.

Suitable retroviral vectors include but are not limited to pLJ, pZip, pWe and pEM, well known to those of skill in the art. Suitable packaging virus lines for replication-defective retroviruses include, for example, ΨCrip, ΨCre, Ψ2 and ΨAm.

Other vectors useful in the present invention include adenovirus, adeno-associated virus, SV40 virus, vaccinia virus, HSV and poxvirus vectors. A preferred vector is the adenovirus. Adenovirus vectors are well known to those skilled in the art and have been used to deliver genes to numerous cell types, including airway epithelium, skeletal muscle, liver, brain and skin (Hitt *et al*, 1997; Anderson, 1998).

A further preferred vector is the adeno-associated (AAV) vector. AAV vectors are well known to those skilled in the art and have been used to stably transduce human T-lymphocytes, fibroblasts, nasal polyp, skeletal muscle, brain, erythroid and haematopoietic stem cells for gene therapy applications. International Patent Application WO 91/18088 describes specific AAV based vectors.

Preferred episomal vectors include transient non-replicating episomal vectors and self-replicating episomal vectors with functions derived from viral origins of replication such as those from EBV, human papovavirus (BK) and BPV-1. Such integrating and episomal vectors are well known to those skilled in the art and are fully described in the body of literature well known to those skilled in the art. In particular, suitable episomal vectors are described in WO98/07876

Mammalian artificial chromosomes can also be used as vectors in the present invention. The use of mammalian artificial chromosomes is discussed by Calos (1996).

In a preferred embodiment, the vector of the present invention is a plasmid. The plasmid may be a non-replicating, non-integrating plasmid.

The term "plasmid" as used herein refers to any nucleic acid encoding an expressible gene and includes linear or circular nucleic acids and double or single stranded nucleic acids. The nucleic acid can be DNA or RNA and may comprise modified nucleotides or ribonucleotides, and may be chemically modified by such means as methylation or the inclusion of protecting groups or cap- or tail structures.

A non-replicating, non-integrating plasmid is a nucleic acid which when transfected into a host cell does not replicate and does not specifically integrate into the host cell's genome (i.e. does not integrate at high frequencies and does not integrate at specific sites).

Highly preferred embodiments of vectors of the invention comprise nucleotides 1 to 1003 and 1747 to 5749 of SEQ ID NO: 2; nucleotides 1 to 9328 and 10072 to 14119 of SEQ ID NO: 3; or nucleotides 1 to 2592 and 3336 to 7383 of SEQ ID NO: 4, being expression vectors suitable for insertion of an expressible sequence in place of the exemplary enhanced green fluorescent protein reporter encoded by the full sequences.

The present invention also provides a host cell transfected with the vector of the present invention. The host cell may be any eukaryotic cell. Preferably it is a mammalian cell, more preferably a human or rodent cell.

Numerous techniques are known and are useful according to the invention for delivering the vectors described herein to cells, including the use of nucleic acid condensing agents, electroporation, complexing with asbestos, polybrene, DEAE cellulose, Dextran, liposomes, cationic liposomes, lipopolyamines, polyornithine, particle bombardment and direct microinjection.

A vector of the invention may be delivered to a host cell non-specifically or specifically. (i.e. to a designated subset of host cells) via a viral or non-viral means of delivery. Preferred delivery methods of viral origin include viral particle-producing packaging cell lines as transfection recipients for the vector of the present invention into which viral packaging signals have been engineered, such as those of adenovirus, herpes viruses and papovaviruses. Preferred non-viral based gene delivery means and methods may also be used in the invention and include direct naked nucleic acid injection, nucleic acid condensing peptides and non-peptides, cationic liposomes and encapsulation in liposomes.

Delivery of a vector according to the invention is contemplated using nucleic acid condensing peptides. Nucleic acid condensing peptides, which are particularly useful for condensing the vector and delivering the vector to a cell, are described in International Patent Application WO 96/41606. Functional groups may be bound to peptides useful for delivery of a vector according to the invention, as described in WO 96/41606. These functional groups may include a ligand that targets a specific cell-type such as a monoclonal antibody, insulin, transferrin, asialoglycoprotein, or a sugar. The ligand thus may target cells in a non-specific manner or in a specific manner that is restricted with respect to cell type.

The functional groups also may comprise a lipid, such as palmitoyl, oleyl, or stearoyl; a neutral hydrophilic polymer such as polyethylene glycol (PEG), or polyvinylpyrrolidine (PVP); a fusogenic peptide such as the HA peptide of influenza virus; or a recombinase or an integrase. The functional group also may comprise an intracellular trafficking protein such as a nuclear localisation sequence (NLS), an endosome escape signal such as a membrane disruptive peptide, or a signal directing a protein directly to the cytoplasm.

The invention further provides a host cell comprising an isolated polynucleotide or vector as herein described. Preferably said cell is a eukaryotic cell, more preferably a mammalian cell, further preferably a human or rodent cell.

In a further aspect, the invention provides a method of expressing an expressible polynucleotide, preferably encoding a polypeptide, comprising inserting an isolated polynucleotide according to the invention into a suitable expression vector as described herein and further inserting said vector into a suitable host cell as described herein and culturing said host cell in suitable conditions to allow expression.

Preferably, said polypeptide is a therapeutically useful polypeptide, preferably selected from the list consisting of an immunoglobulin or a functional epitope-binding fragment of an immunoglobulin, a growth factor, a receptor or soluble fragment thereof and a blood clotting factor.

Also provided is a pharmaceutical preparation comprising a polynucleotide, vector or host cell according to the invention and a pharmaceutically acceptable carrier, excipient, buffer or medium.

## BRIEF DESCRIPTION OF THE DRAWINGS

**Figure 1** shows the nucleotide sequence of the GPCMV IE enhancer/promoter (SEQ ID NO:1). Potential binding sites for the transcription factors AP-1, NF-κB, SRF and GCN4 are shown, together with CAAT and TATA boxes, the CRS initiator site and transcriptional start (arrow).

**Figure 2** shows expression levels obtained with a number of EGFP reporter constructs in CHO-K1 cells, expressed as median fluorescence as measured by FACS. The results compare human and guinea pig CMV IE enhancer/promoters, with and without a 1.5kb hnRNP UCOE element.

**Figure 3** shows a map of reporter plasmid CET 1005 GPCMV-EGFP comprising the GPCMV IE enhancer/promoter (GPCMV) driving expression of an enhanced green fluorescence protein reporter gene (EGFP). The eukaryotic selectable marker is a puromycin resistance gene expressed from a mouse phosphoglycerate kinase promoter. The prokaryotic selectable marker is ampicillin resistance.

**Figure 4** shows a map of reporter plasmid CET1015 8kb-GPCMV-EGFP. This is similar to CET 1005 GPCMV-EGFP with the addition of the 8kb hnRNP UCOE element upstream of the GPCMV IE enhancer/promoter.

**Figure 5** shows a map of reporter plasmid CET1015 1.5kb-GPCMV-EGFP. This is similar to CET 1015 8kb-GPCMV-EGFP with the replacement of the 8kb hnRNP UCOE with a 1.5kb hnRNP UCOE element.

**Figure 6** compares EGFP expression driven by human and guinea pig CMV IE enhanced promoter elements in HEK293 cells (human embryonic kidney cells transformed with sheared adenovirus type 5 DNA)

**Figure 7** shows a similar comparison using luciferase-based reporter constructs.

## DETAILED DESCRIPTION

### Example 1

***Generation of stably transfected CHO-K1 cells using vectors containing either the hCMV promoter or the gpCMV promoter***

The plasmids constructs were generated as follows. The ampicillin resistance gene was isolated from pBluescript® (Stratagene) by PCR incorporating NruI sites within each end of the primer (5'-TGTCGCGAGTCTGACAGTTACCAAT GCTTAATC 3'(SEQ ID NO:5), 5'- CATCGCGAGCACTTTTCGGGGAAATGTGTGCGC-3' (SEQ ID NO:6). The PCR product was inserted into the *Pvu*II site of p*Mae*II (Nucleic Acids Research 2001 29:E26) to generate pCA1. The following oligonucleotides

1. 5'- TCGAAGTTTAAACATTTAAATCTAGAAGCTTAT-3' (SEQ ID NO:7)
2. 5'-CCGGTATCGATAAGCTTCTAGATTTAAATGTTTAAACT-3' (SEQ ID NO:8)
3. 5'-CGATACCGGTGGCGCGCCAATTGTTAATTAAGATCTGG-3' (SEQ ID NO:9)
4. 5'-CCCATTGGGCCAGATCTTAATTAACAATTGGCGCGCCA-3' (SEQ ID NO:10)
5. 5'-CCCAATGGGCCGTACGAATTCCTTAGGCTCGAG-3' (SEQ ID NO:11)
6. 5'-GGCCCTCGAGCCTAAGGAATTCGTACGG-3' (SEQ ID NO:12)

were annealed (1 with 2; 3 with 4; 5 with 6; and then the three dimers were annealed together) and used to replace the multicloning site of pCA1 between the *Xho*I and *Not*I sites destroying these sites during the construction. This generated pCA1MCS. The *Age*I site was deleted from the PGK promoter within pPGK-Puro-bgh pA by *Age*I restriction digestion followed by blunting with T4 DNA polymerise and re-ligation. The PGK-Puromycin pA cassette was removed from this vector as an *Eco*RI-*Xho*I fragment and ligated into pCA1MCS that had similarly been digested with *Eco*RI and *Xho*I. This vector was designated pCIA-Puro (CET 1000). The bghpA in pCIA-Puro was then replaced with the HSV TkpA. The HSV-Tk polyA was removed from pEGFP-N1 as a *Bst*BI-*Eco*109I fragment, blunted with T4 DNA polymerase, and ligated into pCIA-Puro that had been digested with *Sac*I and blunted with T4 DNA polymerase. This vector was designated CET 1005.

To construct pCET1005 1.5kb-GPCMV-EGFP, the 1.5kb hnRNP UCOE fragment was excised from pCET20 (described previously) using *Bsm*BI, blunt-ended using T4 polymerase and then cloned into the blunted *Xho*I site of pEGFP-N1 (Clontech, Palo Alto, CA, USA) generating pEGFP-N1 1.5kb-EGFP. The 2.4kb "hnRNP-EGFP" cassette was then excised from this plasmid using *Nhe*I (blunt-ended)/*Not*I and subcloned into the backbone of pCET1005-EGFP that had been digested with *Swa*I/*Not*I to give pCET1005 1.5kb-EGFP. The GPCMV promoter was then excised from pPCRScript GPCMV (synthesized by Geneart, Regensburg, Germany) with *Nhe*I and *Eco*RI, blunt-ended and subcloned into the blunted *Bam*HI of this plasmid to yield pCET1005 1.5kb-GPCMV-EGFP. Excising the 1.5kb hnRNP UCOE using *Pme*I/*Sac*I, blunt-ending and religating the backbone generated the plasmid pCET1005 GPCMV-EGFP.

To construct pCET1015 8kb-GPCMV-EGFP, the 5.3kb *Sac*I (blunt)/*Pac*I fragment of pCET1005 1.5kb-GPCMV-EGFP was subcloned into the *Asc*I (blunt)/*Pac*I-digested backbone of pCET1015. The plasmid pCET1005 1.5kb-HCMV-EGFP was constructed by subcloning the blunted 1.5kb hnRNP *Bsm*BI fragment from pCET20 into the blunted *Cla*I site of pCET1005-EGFP.

CHO-K1 cells were maintained in F12 (HAM) nutrient mixture (Gibco, UK) supplemented with 10% Foetal Calf Serum (Invitrogen, UK) and 5 U/ml Penicillin and Streptomycin mix (Sigma, UK). For stable transfection of CHO-K1, plasmids were linearised with *Pci*I, extracted in phenol: chloroform: isoamyl alcohol and chloroform, precipitated in ethanol and resuspended at a concentration of 0.25μg/μl in sterile water. In a sterile electroporation cuvette, equivalent molar quantities of linearised plasmids were diluted to 25μl in sterile water (1.39μg pCET1005-EGFP, 1.78μg pCET1005 1.5kb-HCMV-EGFP, 1.45μg pCET1005 GPCMV-EGFP or 1.85μg pCET1005 1.5kb-GPCMV-EGFP) and mixed with $5x10^6$ CHO-K1 cells in 250μl growth medium. After incubation on ice for 15 minutes, the cells were electroporated at 250V/975μF (BioRad Gene Pulser II™) and incubated at room temperature for a further 10 minutes. Cells were then transferred into 10ml of growth media, harvested by centrifugation and transferred into a $225cm^2$ tissue culture flask in a total of 50ml of growth medium. Cells were incubated for 24 hours at 37°C in a 5% $CO_2$ incubator before addition of Puromycin (Sigma, UK) to a concentration of 12.5μg/ml. Cells were cultured for 8 days (replacing selective media after 4 days) before the stable transfectants were harvested, subcultured in 6-well tissue culture dishes (maintaining selection) and analysed by Fluorescence Activated Cell Sorting using the FL1 channel to view EGFP. Figure 2 clearly shows that the two gpCMV containing constructs pCET1005-GPCMV-EGFP (Figure 3) and pCET1005-1.5kb-GPCMV-EGFP (Figure 5) generate pools which express the transgene to a higher level than the corresponding constructs which use the hCMV promoter, pCET1005-EGFP and pCET1005-1.5kb-HCMV-EGFP respectively.

**Example 2**

HEK293 cells were maintained in Dulbecco's Modified Eagle Medium (DMEM; Sigma, UK) supplemented with 10% Foetal Calf Serum and 5 U/ml Penicillin and Streptomycin mix. For stable transfection, HEK293 cells were seeded into 6-well dishes at a density of $1x10^6$ cells/well and cultured for 24 hours at 37°C in a 5% $CO_2$ incubator. Cells were then transfected with 4μg of the indicated plasmid (pCET1005-EGFP or pCET1005-gpCMV-EGFP)(linearised with *Pci*I) using 10μl Lipofectamine 2000 (Invitrogen, UK).

The DNA and Lipofectamine 2000 were diluted separately in 250μl OptiMEM I (Gibco, UK) and, after incubation at room temperature for 5 minutes, mixed together and incubated for a further 20 minutes. Growth media on the cells was replaced with 1ml of OptiMEM I supplemented with 15% FCS and the DNA/Lipofectamine 2000 mixture was then added. Cells were incubated at 37°C in a 5% $CO_2$ incubator for 5 hours before 3.5ml of OptiMEM I supplemented with 10% FCS was added. Cells were then incubated at 37°C in a 5% $CO_2$ incubator for 24 hours before being harvested and transferred to a $225cm^2$ tissue culture flask in a total of 50ml of DMEM growth medium, supplemented with 0.5μg/ml Puromycin. Cells were grown for approximately 14 days (replacing the selective media every 3-4 days) before the stable transfectants were harvested by centrifugation, subcultured in 6-well tissue culture dishes (maintaining selection) and analysed by Fluorescence Activated Cell Sorting using the FL1 channel to view EGFP.
Figure 6 shows that the pools generated with the gpCMV construct give EGFP expression levels three to four fold higher than those generated with the hCMV construct.

**Example 3**

CHO-K1 cells were cultured as described for Example 1. $1.5x10^5$ CHO-K1 cells were seeded 24 hrs before transfection into 12-wells. 24 hrs later, cells were transfected with 1 ug Luciferase reporter plasmid (phCMV-Luc or pgpCMV-Luc) using 1.5 ul FUGENE (Roche, UK). For this, FUGENE and DNA were both diluted separately in Opti-MEM I (Invitrogen), mixed together and incubated for 30 min at RT before added to the cells. Luciferase expression was analysed 24 hrs later using a Berthold luminometer (Berthold, Wildbad, Germany). Generally, cell lysis and luciferase reporter assay were performed as described earlier (Lipinski et al., Gene Therapy, 2001 (8): 274-281). Transfections were done in triplicate and the mean and standard deviation of one representative experiment are shown (Figure 7). Clearly the gpCMV vector was at least two-fold more active luciferase than the hCMV plasmid.

The plasmid hCMV-Luc has been described earlier (Lipinski et al., Gene Therapy (2001) 8:274-281). The plasmid gpCMV-Luc was generated by preparing a *Nde*I/*Eco*RI fragment from pCRScript/gpCMV (customer gene synthesis company: Geneart, Regensburg, Germany) and cloning this gpCMV promoter fragment into the blunted *Xho*I site of pGL3basic (Promega).

## Claims

1. An isolated polynucleotide comprising

   a. an element comprising at least 200 contiguous nucleotides of SEQ ID NO:1, wherein said 200 contiguous nucleotides comprise a promoter and/or an enhancer element and
   b. an element comprising an expressible polynucleotide sequence;

   **characterised in that** said isolated polynucleotide comprises, in a 5' to 3' direction with respect to the sense strand of the expressible polynucleotide sequence, an enhancer, a single promoter, and said expressible polynucleotide sequence, and wherein said enhancer is operably linked to said promoter, which is directly operably linked to said expressible polynucleotide sequence and wherein said promoter is not naturally operably linked to said expressible polynucleotide sequence.

2. An isolated polynucleotide according to claim 1, comprising at least 500 contiguous nucleotides of SEQ ID NO:1

3. An isolated polynucleotide according to either of claim 1 or 2, comprising nucleotides 50 to 550 of SEQ ID NO:1

4. An isolated polynucleotide according to either of claim 1 or 2, comprising nucleotides 275 to 775 of SEQ ID NO:1

5. An isolated polynucleotide according to either of claim 1 or 2, comprising the promoter from the immediate/early regulatory region of guinea pig CMV directly operably linked to an expressible nucleic acid sequence to which it is not naturally operably linked.

6. An isolated polynucleotide according to claim 5, comprising nucleotide 679 to 880 of SEQ ID NO: 1.

7. An isolated polynucleotide according to any preceding claim, comprising nucleotides 1 to 887 of SEQ ID NO: 1

8. An isolated polynucleotide according to any preceding claim, further comprising an extended, methylation-free CpG island operably linked to said expressible nucleic acid sequence.

9. A vector comprising the polynucleotide of any preceding claim.

10. A eukaryotic expression vector according to claim 9.

11. A vector according to either claim 9 or claim 10, comprising the polynucleotide sequence of nucleotides 1 to 1003 and 1747 to 5749 of SEQ ID NO: 2.

12. A vector according to either claim 9 or claim 10, comprising nucleotides 1 to 9328 and 10072 to 14119 of SEQ ID NO: 3.

13. A vector according to either claim 9 or claim 10, comprising nucleotides 1 to 2592 and 3336 to 7383 of SEQ ID NO: 4.

14. A host cell comprising an isolated polynucleotide according to any of claims 1 to 5, or the vector of any of claims 11 to 13.

15. A method of expressing a polypeptide comprising inserting an expressible nucleic acid sequence encoding said polypeptide into an expression vector according to any of claims 11 to 13 and further inserting said vector into an appropriate host cell and culturing said host cell in suitable conditions to allow expression.

16. Method according to claim 15, wherein said polypeptide is a therapeutically useful polypeptide.

**17.** Method according to claim 16, wherein said polypeptide is selected from the list consisting of: an immunoglobulin, a functional epitope-binding fragment of an immunoglobulin, a growth factor, soluble receptor and blood clotting factor.

**18.** A pharmaceutical preparation comprising a polynucleotide according to any of claims 1 to 8, a vector according to any of claims 11 to 13, or a host cell according to claim 14 and a pharmaceutically acceptable carrier, excipient, buffer or medium.

## Patentansprüche

**1.** Isoliertes Polynukleotid, das Folgendes umfasst;

a. ein Element, das mindestens 200 benachbarte Nukleotide von SEQ ID NO: 1 umfasst, wobei die 200 benachbarten Nukleotide einen Promotor und/oder ein Enhancer-Element umfassen, und
b. ein Element, das eine exprimierbare Polynukleotidsequenz umfasst,

**dadurch gekennzeichnet, dass** das isolierte Polynukleotid in einer 5'-zu-3'-Richtung in Bezug auf den Sense-Strang der exprimierbaren Polynukleotidsequenz einen Enhancer, einen einzigen Promotor und die exprimierbare Polynukleotidsequenz umfasst und wobei der Enhancer mit dem Promotor operativ verknüpft ist, der direkt mit der exprimierbaren Polynukleotidsequenz operativ verknüpft ist, und wobei der Promotor nicht naturgemäß mit der exprimierbaren Polynukleotidsequenz operativ verknüpft ist.

**2.** Isoliertes Polynukleotid nach Anspruch 1, das mindestens 500 benachbarte Nukleotide von SEQ ID NO: 1 umfasst,

**3.** Isoliertes Polynukleotid nach Anspruch 1 oder 2, das die Nukleotiden 50 bis 550 von SEQ ID NO: 1 umfasst.

**4.** Isoliertes Polynukleotid nach Anspruch 1 oder 2, das die Nukleotiden 275 bis 775 von SEQ ID NO: 1 umfasst.

**5.** Isoliertes Polynukleotid nach Anspruch 1 oder 2, das den Promotor aus der Immediate-Early-Regulationsregion des Meerschweinchen-CMV umfasst, der direkt mit einer exprimierbaren Nukleinsäuresequenz operativ verknüpft ist, mit der er nicht naturgemäß operativ verknüpft ist.

**6.** Isoliertes Polynukleotid nach Anspruch 5, das die Nukleotiden 679 bis 880 von SEQ ID NO:1 umfasst.

**7.** Isoliertes Polynukleotid nach einem der vorhergehenden Ansprüche, das die Nukleotiden 1 bis 887 von SEQ ID NO: 1 umfasst.

**8.** Isoliertes Polynukleotid nach einem der Ansprüche, das weiterhin eine erweiterte, methylierungsfreie CpG-Insel umfasst, die mit der exprimierbaren Nukleinsäuresequenz operativ verknüpft ist.

**9.** Vektor, der das Polynukleotid nach einem der vorhergehenden Ansprüche umfasst.

**10.** Eukaryontischer Expressionsvektor nach Anspruch 9.

**11.** Vektor nach Anspruch 9 oder 10, der die Polynukleotidsequenz der Nukleotide I bis 1003 und 1747 bis 5749 von SEQ ID NO: 2 umfasst.

**12.** Vektor nach Anspruch 9 oder 10, der die Nukleotide 1 bis 9328 und 10.072 bis 14.119 von SEQ 117 NO: 3 umfasst.

**13.** Vektor nach Anspruch 9 oder 10, der die Nukleotide 1 bis 2592 und 3336 bis 7383 von SEQ ID NO: 4 umfasst.

**14.** Wirtszelle, die ein isoliertes Polynukleotid nach einem der Ansprüche 1 bis 5 oder den Vektor nach einem der Ansprüche 11 bis 13 umfasst.

**15.** Verfahren zur Expression eines Polypeptids, das das Insertieren einer exprimierbaren Nukleinsäuresequenz, die das Polypeptid codiert, in einen Expressionsvektor nach einem der Ansprüche 11 bis 13 und weiterhin das Insertieren des Vektors in eine adäquate Wirtszelle und das Kultivieren der Wirtszelle in geeigneten Bedingungen, die eine Expression ermöglichen, umfasst.

**16.** Verfahren nach Anspruch 15, wobei das Polypeptid ein therapeutisch geeignetes Polypeptid ist,

**17.** Verfahren nach Anspruch 16, wobei das Polypeptid aus der Liste ausgewählt ist, die aus Folgendem besteht: einem Immunglobulin, einem funktionellen epitopbindenden Fragment eines Immunglobulins, einem Wachstumsfaktor, einem löslichen Rezeptor und einem Blutgerinnungsfaktor.

**18.** Pharmazeutisches Präparat, das ein Polynukleotid nach einem der Ansprüche I bis 8, einen Vektor nach einem der Ansprüche 11 bis 13 oder eine Wirtszelle nach Anspruch 14 und einen pharmazeutisch unbedenklichen Trägerstoff, ein pharmazeutisch unbedenkliches Vehikel, einen pharmazeutisch unbedenklichen Puffer oder ein pharmazeutisch unbedenkliches Medium umfasst.

## Revendications

**1.** Polynucléotide isolé comprenant

a. un élément comprenant au moins 200 nucléotides contigus de SEQ ID N° ; 1, dans lequel lesdits 200 nucléotides contigus comprennent un promoteur et/ou un élément amplificateur et
b. un élément comprenant une séquence polynucléotidique exprimable ;

**caractérisé en ce que** ledit polynucléotide isolé comprend, dans un sens 5' vers 3' par rapport au brin sens de la séquence polynucléotidique exprimable, un amplificateur, un promoteur unique et ladite séquence polynucléotidique exprimable, et dans lequel ledit amplificateur est lié de manière opérationnelle audit promoteur qui est directement lié de manière opérationnelle à ladite séquence polynucléotidique exprimable et dans lequel ledit promoteur n'est pas naturellement lié de manière opérationnelle à ladite séquence polynucléotidique exprimable.

**2.** Polynucléotide isolé selon la revendication 1, comprenant au moins 500 nucléotides contigus de SEQ ID N° : 1.

**3.** Polynucléotide isolé selon la revendication 1 ou 2, comprenant les nucléotides 50 à 550 de SEQ ID N°: 1.

**4.** Polynucléotide isolé selon la revendication 1 ou 2, comprenant les nucléotides 275 à 775 de SEQ ID N°: 1.

**5.** Polynucléotide isolé selon la revendication 1 ou 2, comprenant le promoteur provenant de la région régulatrice immédiate/précoce du CMV de cobaye directement lié de manière opérationnelle à une séquence d'acide nucléique exprimable à laquelle il n'est pas naturellement lié de manière opérationnelle.

**6.** Polynucléotide isolé selon la revendication 5, comprenant les nucléotides 679 à 880 de SEQ ID N° :1.

**7.** Polynucléotide isolé selon l'une quelconque des revendications précédentes, comprenant les nucléotides 1 à 887 de SEQ ID N° : 1.

**8.** Polynucléotide isolé selon l'une quelconque des revendications précédentes, comprenant en outre un îlot CpG étendu, exempt de méthylation lié de manière opérationnelle à ladite séquence d'acide nucléique exprimable.

**9.** Vecteur comprenant le polynucléotide selon l'une quelconque des revendications précédentes.

**10.** Vecteur d'expression eucaryote selon la revendication 9.

**11.** Vecteur selon la revendication 9 ou la revendication 10, comprenant la séquence polynucléotidique de nucléotides 1 à 1003 et 1747 à 5749 de SEQ ID N° : 2.

**12.** Vecteur selon la revendication 9 ou la revendication 10, comprenant les nucléotides 1 à 9328 et 10072 à 14119 de SEQ ID N°: 3.

**13.** Vecteur selon la revendication 9 ou la revendication 10, comprenant les nucléotides 1 à 2592 et 3336 à 7383 de SEQ ID N° : 4.

**14.** Cellule hôte comprenant un polynucléotide isolé selon l'une quelconque des revendications 1 à 5, ou le vecteur selon l'une quelconque des revendications 11 à 13.

**15.** Procédé d'expression d'un polypeptide comprenant l'insertion d'une séquence d'acide nucléique exprimable codant pour ledit polypeptide dans un vecteur d'expression selon l'une quelconque des revendications 11 à 13 et l'insertion supplémentaire dudit vecteur dans une cellule hôte appropriée et la mise en culture de ladite cellule hôte dans des conditions appropriées pour permettre l'expression.

**16.** Procédé selon la revendication 15, dans lequel ledit polypeptide est un polypeptide utile du point de vue thérapeutique.

**17.** Procédé selon la revendication 16, dans lequel ledit polypeptide est sélectionné parmi la liste constituée de : une immunoglobuline, un fragment fonctionnel de liaison à un déterminant antigénique d'une immunoglobuline, un facteur de croissance, un récepteur soluble et un facteur de coagulation sanguine.

**18.** Préparation pharmaceutique comprenant un polynucléotide selon l'une quelconque des revendications 1 à 8, un vecteur selon l'une quelconque des revendications 11 à 13 ou une cellule hôte selon la revendication 14 et un support, excipient, tampon ou milieu pharmaceutiquement acceptable.

# Figure 1

```
AP1                                     NFκB
ttagtcatatgttacttggcagaggccgcatggaaagtccctggacgtgg   50
                                                  SRF
gacatctgattaatacgtgaggaggtcagccatgttcttttggcaaagg   100

actacggtcattggacgtttgattggcatgggatagggtcagccagagtt   150
          SRF                      NFκB
aacagtgttcttttggcaaagggatacgtggaaagtcccgggccatttac   200
                                             AP1
agtaaactgatacggggacaaagcacagccatatttagtcatgtattgct   250
                     NFκB
tggcagagggtctatggaaagtccctggacgtgggacgtctgattaatat   300
                                          SRF
gaaagaaggtcagccagaggtagctgtgtccttttggcaaagggatacg   350

gttatgggacgtttgattggactgggatagggtcagccagagttaacagt   400
   SRF                    NFκB
gttcttttggcaaaggaaacgtggaaagtcccgggccatttacagtaaac   450
                                  AP1       SRF
tgatactgggacaaagtacacccatatttagtcatgttcttttggcaaa   500
          NFκB                GCN4
gagcatctggaaagtcccgggcagcattatagtcacttggcagagggaaa   550

gggtcactcagagttaagtacatctttccagggccaatattccagtaaat   600
              AP1                           NFκB
tacacttagtttatgcaaatcagccacaaaggggatttcccggtcaat   650
GCN4            AP1           CAAT box
tatgactttttccttagtcatgcggtatccaattactgccaaattggcag   700
                         GCN4                 NFκB
tacatactaggtgattcactgacatttggccgtcctctggaaagtccctg   750
                                GCN4
gaaaccgctcaagtactgtatcatggtgactttgcattttggagagcac   800

gccccactccaccattggtccacgtaccctatgggggagtggtttatgag   850
TATA Box          CRS
tatataagggctccggtttagaagccgggcagagcg                 887
```

Figure 2

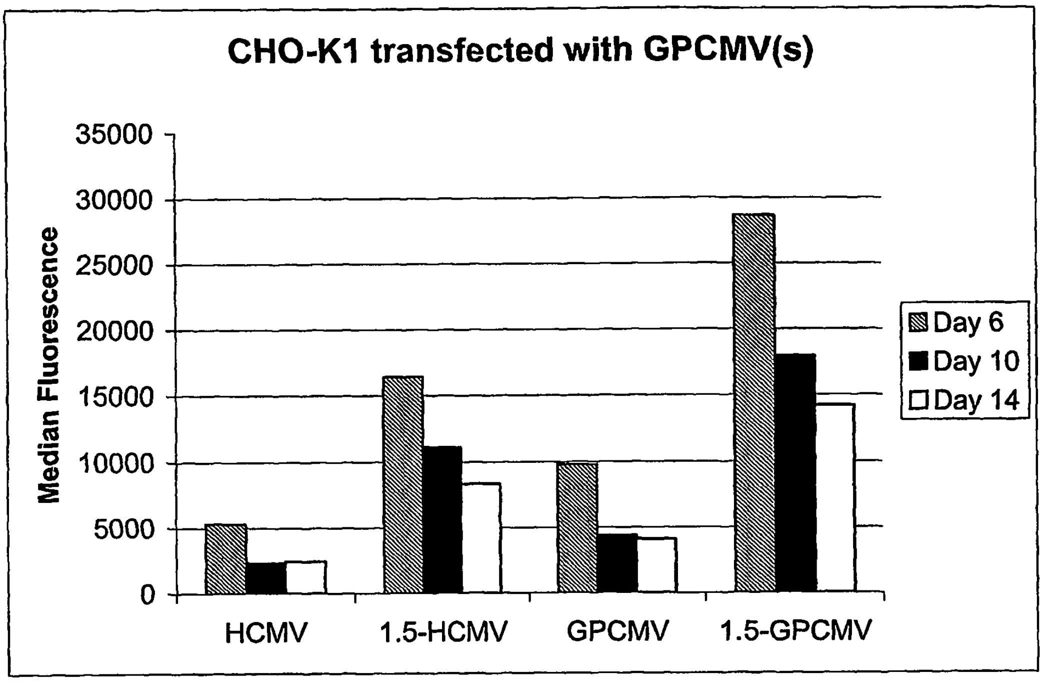
CHO-K1 transfected with GPCMV(s)
Median Fluorescence
35000
30000
25000
20000
15000
10000
5000
0
HCMV
1.5-HCMV
GPCMV
1.5-GPCMV
Day 6
Day 10
Day 14

# Figure 3

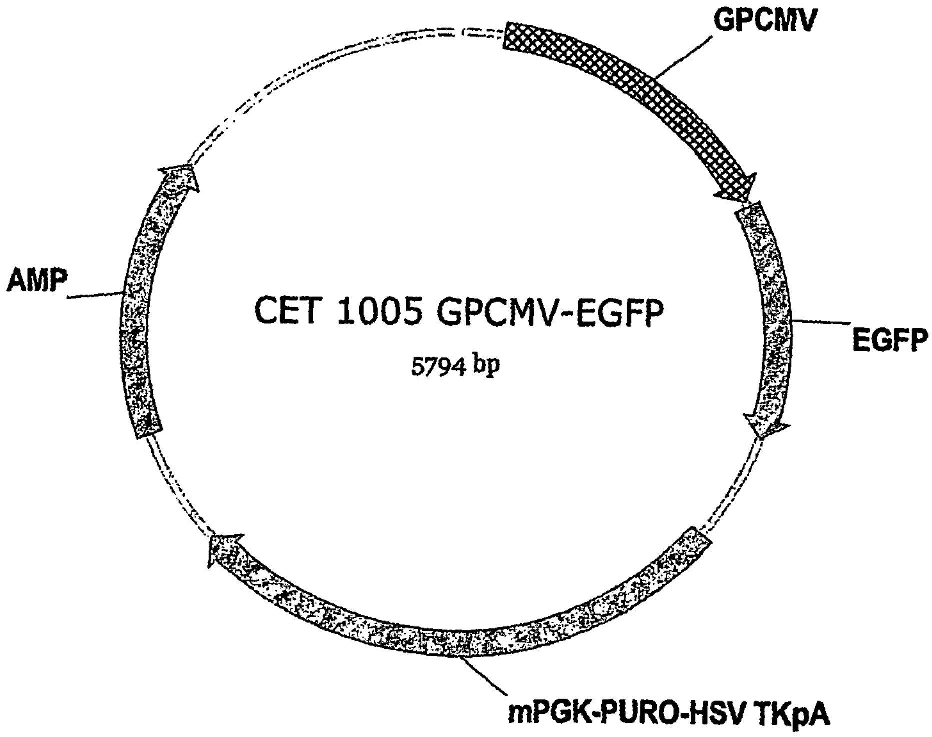

# Figure 4

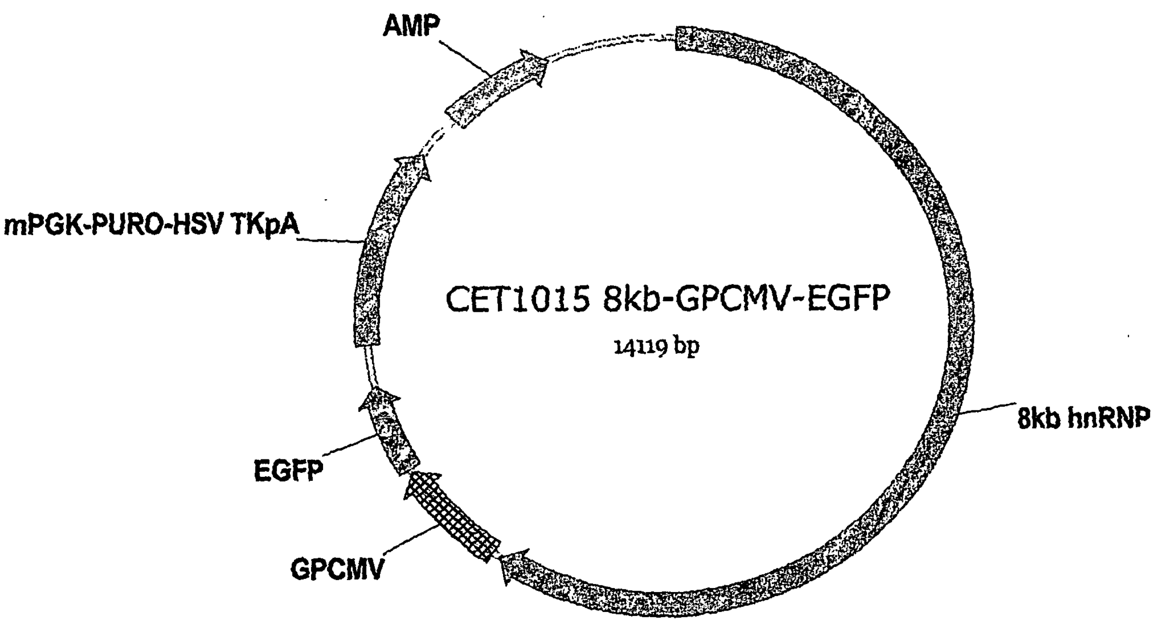
AMP
mPGK-PURO-HSV TKpA
CET1015 8kb-GPCMV-EGFP
14119 bp
8kb hnRNP
EGFP
GPCMV

# Figure 5

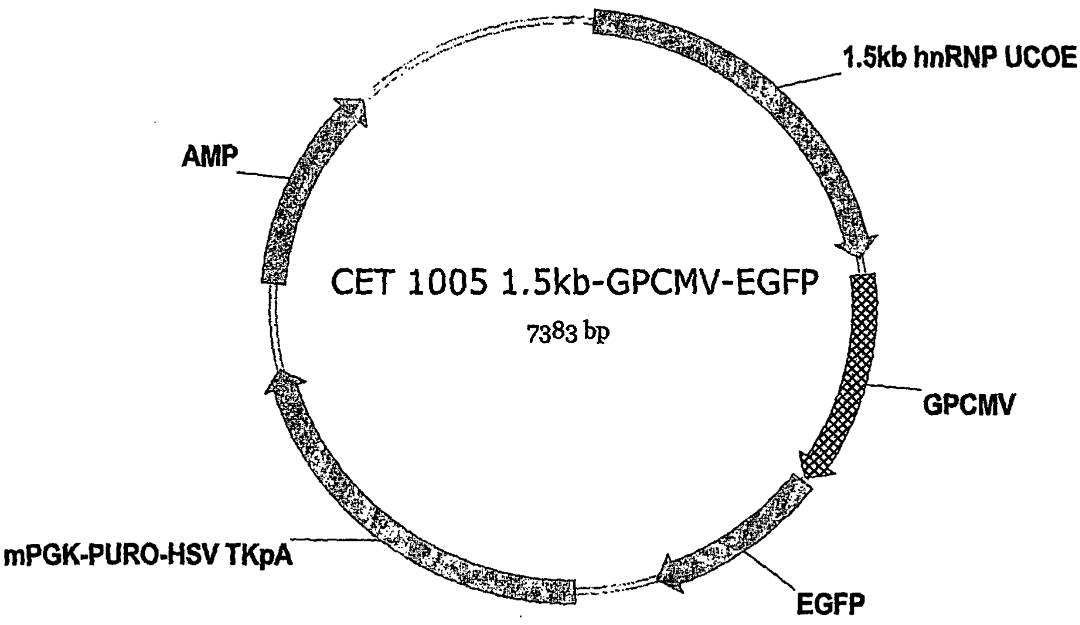
1.5kb hnRNP UCOE
AMP
CET 1005 1.5kb-GPCMV-EGFP
7383 bp
GPCMV
mPGK-PURO-HSV TKpA
EGFP

**Figure 6**

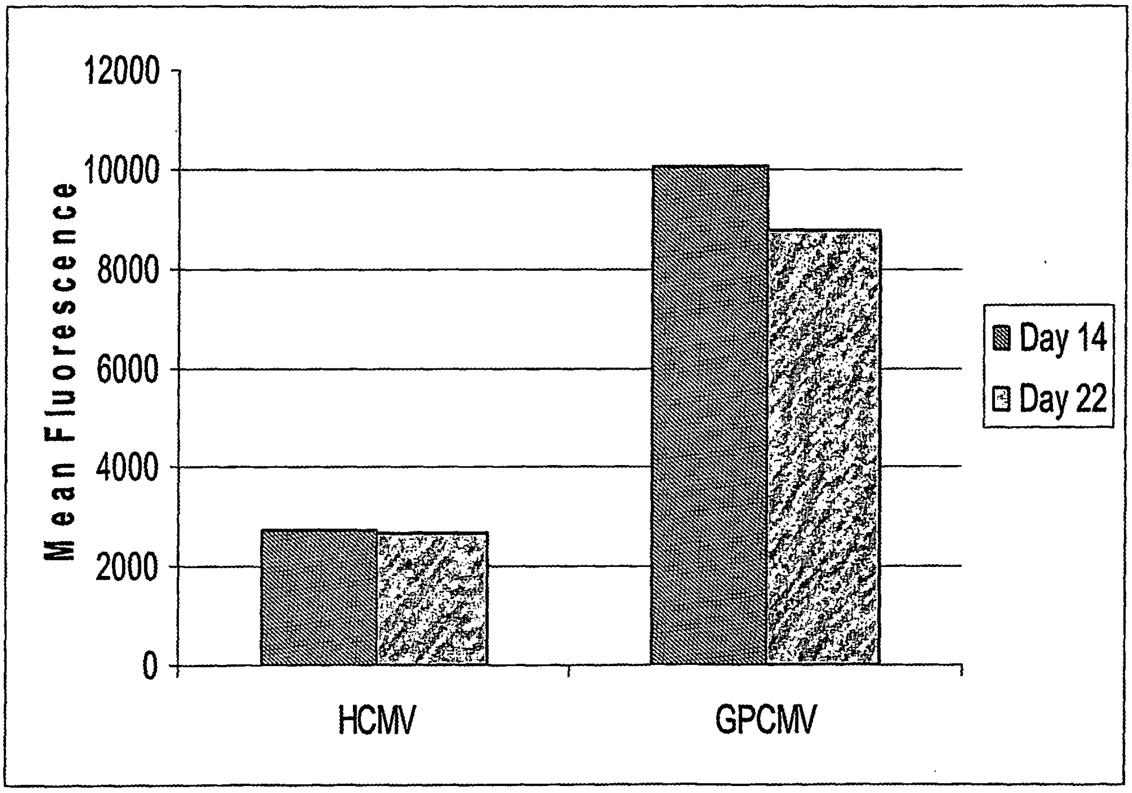
12000
10000
8000
6000
4000
2000
0
Mean Fluorescence
HCMV
GPCMV
Day 14
Day 22

Figure 7

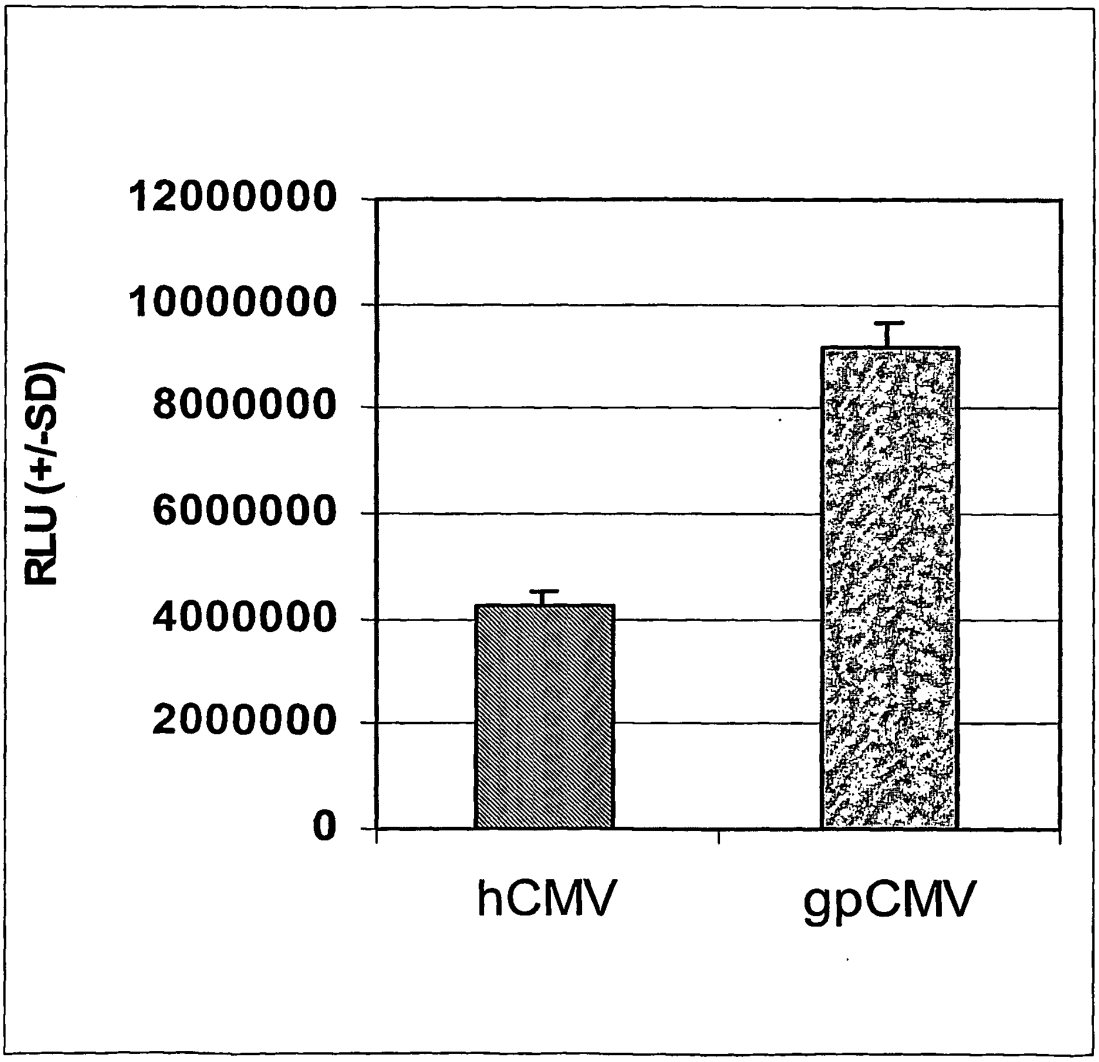

12000000
10000000
8000000
6000000
4000000
2000000
0
RLU (+/-SD)
hCMV
gpCMV

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5168062 A
- US 5591639 A
- US 4968615 A, Koszinowski
- GB 9902357 W
- WO 0005393 A
- WO 0224930 A
- WO 04067701 A
- WO 9118088 A
- WO 9807876 A
- WO 9641606 A


### Non-patent literature cited in the description


- **McKnight ; Tjian.** *Cell,* 1987, vol. 46, 795-805
- **Breathnach ; Chambon.** *Annu Rev Biochem,* 1981, vol. 50, 349-393
- **Kadonaga.** *Cell,* 2004, vol. 116, 247-257
- **Ondek et al.** *Science,* 1988, vol. 236, 1237-1244
- **Foecking ; Hoffstetter.** *Gene,* 1986, vol. 45, 101-105
- **Isomura ; Stinski.** *J Virol,* 2003, vol. 77, 3602-3614
- **Castillo ; Kowalik.** *Gene,* 2002, vol. 290, 19-34
- **Thomsen et al.** *Proc Natl Acad Sci USA,* 1984, vol. 81, 659-663
- **Meier ; Stinski.** *Intervirology,* 1996, vol. 39, 331-342
- **Dorsch-Hasler et al.** *Proc Natl Acad Sci USA,* 1985, vol. 82, 8325-8329
- **Isom et al.** *J Virology,* 1984, vol. 49, 426-436
- **Gao ; Isom.** *J Virology,* 1984, vol. 52, 436-447
- **Yin et al.** *J Virol,* 1990, vol. 64, 1537-1548
- **Yin.** Guinea pig cytomegalovirus immediate-early gene expression. *PhD thesis,* 1991
- **Hill et al.** *Science,* 1984, vol. 234, 451-457
- **Gardiner-Green M ; Frommer M.** *J Mol Biol,* 1987, vol. 196, 261-282
- **Rice P ; Longden I ; Bleasby A.** *Trends Genet,* 2000, vol. 16, 276-277
- **Bird et al.** *Cell,* 1985, vol. 40, 91-99
- **Tazi ; Bird.** *Cell,* 1990, vol. 60, 909-920
- **Wise ; Pravtcheva.** *Genomics,* 1999, vol. 60, 258-271
- **Antequera, F. ; Bird, A.** *Proc. Natl. Acad. Sci. USA,* 1993, vol. 90, 1195-11999
- **Cross, S.H. ; Bird, A.P.** *Curr. Opin, Genet. Dev.,* 1995, vol. 5, 309-314
- **Tazi, J. ; Bird, A.** *Cell,* 1990, vol. 60, 909-920
- **Sambrook et al.** Molecular Cloning; A Laboratory Approach. 1989
- *Nucleic Acids Research,* 2001, vol. 29, E26
- **Lipinski et al.** *Gene Therapy,* 2001, 274-281
- **Lipinski et al.** *Gene Therapy,* 2001, vol. 8, 274-281

SEQUENCE LISTING

<110> ML Laboratories PLC

<120> Vectors comprising guinea pig CMV promoter

<130> P108419GB

<160> 12

<170> PatentIn version 3.2

<210> 1
<211> 887
<212> DNA
<213> Guinea pig cytomegalovirus

<400> 1

```
ttagtcatat gttacttggc agaggccgca tggaaagtcc ctggacgtgg gacatctgat      60
taatacgtga ggaggtcagc catgttcttt ttggcaaagg actacggtca ttggacgttt     120
gattggcatg ggatagggtc agccagagtt aacagtgttc ttttggcaaa gggatacgtg     180
gaaagtcccg ggccatttac agtaaactga tacggggaca aagcacagcc atatttagtc     240
atgtattgct tggcagaggg tctatggaaa gtccctggac gtgggacgtc tgattaatat     300
gaaagaaggt cagccagagg tagctgtgtc ctttttggca aagggatacg gttatgggac     360
gtttgattgg actgggatag ggtcagccag agttaacagt gttcttttgg caaaggaaac     420
gtggaaagtc ccgggccatt tacagtaaac tgatactggg acaaagtaca cccatattta     480
gtcatgttct ttttggcaaa gagcatctgg aaagtcccgg gcagcattat agtcacttgg     540
cagagggaaa gggtcactca gagttaagta catctttcca gggccaatat tccagtaaat     600
tacacttagt tttatgcaaa tcagccacaa aggggatttt cccggtcaat tatgactttt     660
tccttagtca tgcggtatcc aattactgcc aaattggcag tacatactag gtgattcact     720
gacatttggc cgtcctctgg aaagtccctg gaaaccgctc aagtactgta tcatggtgac     780
tttgcatttt tggagagcac gccccactcc accattggtc cacgtaccct atgggggagt     840
ggtttatgag tatataaggg gctccggttt agaagccggg cagagcg                   887
```

<210> 2
<211> 5794
<212> DNA
<213> Artificial

<220>
<223> Eukaryotic expression vector

<400> 2

```
cgttgtaaaa cgacggccag tgaattgtaa tacgactcac tatagggcga attgggtacc      60
gggccccccc tcgaagtttc aagcttcgaa ttctgcagtc gacggtaccg cgggcccggg     120
atctatgtta cttggcagag gccgcatgga aagtccctgg acgtgggaca tctgattaat     180
acgtgaggag gtcagccatg ttctttttgg caaaggacta cggtcattgg acgtttgatt     240
ggcatgggat agggtcagcc agagttaaca gtgttctttt ggcaaaggga tacgtggaaa     300
```

```
gtcccgggcc atttacagta aactgatacg gggacaaagc acagccatat ttagtcatgt    360
attgcttggc agagggtcta tggaaagtcc ctggacgtgg gacgtctgat taatatgaaa    420
gaaggtcagc cagaggtagc tgtgtccttt ttggcaaagg gatacggtta tgggacgttt    480
gattggactg ggatagggtc agccagagtt aacagtgttc ttttggcaaa ggaaacgtgg    540
aaagtcccgg gccatttaca gtaaactgat actgggacaa agtacaccca tatttagtca    600
tgttcttttt ggcaaagagc atctggaaag tcccgggcag cattatagtc acttggcaga    660
gggaaagggt cactcagagt taagtacatc tttccagggc caatattcca gtaaattaca    720
cttagtttta tgcaaatcag ccacaaaggg gattttcccg gtcaattatg actttttcct    780
tagtcatgcg gtatccaatt actgccaaat tggcagtaca tactaggtga ttcactgaca    840
tttggccgtc ctctggaaag tccctggaaa ccgctcaagt actgtatcat ggtgactttg    900
catttttgga gagcacgccc cactccacca ttggtccacg taccctatgg gggagtggtt    960
tatgagtata taaggggctc cggtttagaa gccgggcaga gcggaattga tccaccggtc   1020
gccaccatgg tgagcaaggg cgaggagctg ttcaccgggg tggtgcccat cctggtcgag   1080
ctggacggcg acgtaaacgg ccacaagttc agcgtgtccg gcgagggcga gggcgatgcc   1140
acctacggca agctgaccct gaagttcatc tgcaccaccg gcaagctgcc cgtgccctgg   1200
cccaccctcg tgaccaccct gacctacggc gtgcagtgct tcagccgcta ccccgaccac   1260
atgaagcagc acgacttctt caagtccgcc atgcccgaag gctacgtcca ggagcgcacc   1320
atcttcttca aggacgacgg caactacaag acccgcgccg aggtgaagtt cgagggcgac   1380
accctggtga accgcatcga gctgaagggc atcgacttca aggaggacgg caacatcctg   1440
gggcacaagc tggagtacaa ctacaacagc cacaacgtct atatcatggc cgacaagcag   1500
aagaacggca tcaaggtgaa cttcaagatc cgccacaaca tcgaggacgg cagcgtgcag   1560
ctcgccgacc actaccagca gaacaccccc atcggcgacg gccccgtgct gctgcccgac   1620
aaccactacc tgagcaccca gtccgccctg agcaaagacc ccaacgagaa gcgcgatcac   1680
atggtcctgc tggagttcgt gaccgccgcc gggatcactc tcggcatgga cgagctgtac   1740
aagtaaagcg gccgcgactc tagatcataa tcagccatac cacatttgta gaggttttac   1800
ttgctttaaa aaacctccca cacctccccc tgaacctgaa acataaaatg aatgcaattg   1860
ttgttgttaa cttgtttatt gcagcttata atggttacaa ataaagcaat agcatcacaa   1920
atttcacaaa taaagcattt ttttcactgc attctagttg tggtttgtcc aaactcatca   1980
atgtatctta actagagtcg acctgcaggc atgcaagctt accggtggcg cgcgcgccaa   2040
ttgttaatta agatctggcc caatgggccg tacgaattcc ttaggctacc gggtagggga   2100
ggcgcttttc ccaaggcagt ctggagcatg cgctttagca gccccgctgg gcacttggcg   2160
ctacacaagt ggcctctggc ctcgcacaca ttccacatcc accggccggt aggcgccaac   2220
cggctccgtt ctttggtggc cccttcgcgc caccttctac tcctccccta gtcaggaagt   2280
tcccccccgc cccgcagctc gcgtcgtgca ggacgtgaca aatggaagta gcacgtctca   2340
```

ctagtctcgt gcagatggac agcaccgctg agcaatggaa gcgggtaggc ctttggggca 2400
gcggccaata gcagctttgc tccttcgctt tctgggctca gaggctggga aggggtgggt 2460
ccgggggcgg gctcaggggc gggctcaggg gcggggcggg cgcccgaagg tcctccggag 2520
gcccggcatt ctgcacgctt caaaagcgca cgtctgccgc gctgttctcc tcttcctcat 2580
ctccgggcct ttcgaccagc ttaccatgac cgagtacaag cccacggtgc gcctcgccac 2640
ccgcgacgac gtccccaggg ccgtacgcac cctcgccgcc gcgttcgccg actaccccgc 2700
cacgcgccac accgtcgatc cggaccgcca catcgagcgg gtcaccgagc tgcaagaact 2760
cttcctcacg cgcgtcgggc tcgacatcgg caaggtgtgg gtcgcggacg acggcgccgc 2820
ggtggcggtc tggaccacgc cggagagcgt cgaagcgggg gcggtgttcg ccgagatcgg 2880
cccgcgcatg gccgagttga gcggttcccg gctggccgcg cagcaacaga tggaaggcct 2940
cctggcgccg caccggccca aggagcccgc gtggttcctg gccaccgtcg gcgtctcgcc 3000
cgaccaccag ggcaagggtc tgggcagcgc cgtcgtgctc cccggagtgg aggcggccga 3060
gcgcgccggg gtgcccgcct tcctggagac ctccgcgccc cgcaacctcc ccttctacga 3120
gcggctcggc ttcaccgtca ccgccgacgt cgaggtgccc gaaggaccgc gcacctggtg 3180
catgacccgc aagcccggtg cctgacgccc gccccacgac ccgcagcgcc cgaccgaaag 3240
gagcgcacga ccccatgcat cgtagacgaa atgaccgacc aagcgacgcc caacctgcca 3300
tcacgagatt tcgattccac cgccgccttc tatgaaaggt tgggcttcgg aatcgttttc 3360
cgggacgccg gctggatgat cctccagcgc ggggatctca tgctggagtt cttcgcccac 3420
cctaggggga ggctaactga aacacggaag gagacaatac cggaaggaac ccgcgctatg 3480
acggcaataa aaagacagaa taaaacgcac ggtgttgggt cgtttgttca taaacgcggg 3540
gttcggtccc agggctggca ctctgtcgat accccaccga gaccccattg gggccaatac 3600
gcccgcgttt cttccttttc cccaccccac cccccaagtt cgggtgaagg cccagggctc 3660
gcagccaacg tcggggcggc aggccccccag cttttgttcc ctttagtgag ggttaatttc 3720
gagcttggcg taatcatggt catagctgtt tcctgtgtga aattgttatc cgctcacaat 3780
tccacacaac atacgagccg gaagcataaa gtgtaaagcc tggggtgcct aatgagtgag 3840
ctaactcaca ttaattgcgt tgcgctcact gcccgctttc cagtcgggaa acctgtcgtg 3900
ccagcatcgc gagcactttt cggggaaatg tgcgcggaac ccctatttgt ttatttttct 3960
aaatacattc aaatatgtat ccgctcatga gacaataacc ctgataaatg cttcaataat 4020
attgaaaaag gaagagtatg agtattcaac atttccgtgt cgcccttatt cccttttttg 4080
cggcattttg ccttcctgtt tttgctcacc cagaaacgct ggtgaaagta aaagatgctg 4140
aagatcagtt gggtgcacga gtgggttaca tcgaactgga tctcaacagc ggtaagatcc 4200
ttgagagttt tcgccccgaa gaacgttttc caatgatgag cacttttaaa gttctgctat 4260
gtggcgcggt attatcccgt attgacgccg ggcaagagca actcggtcgc cgcatacact 4320
attctcagaa tgacttggtt gagtactcac cagtcacaga aaagcatctt acggatggca 4380

```
tgacagtaag agaattatgc agtgctgcca taaccatgag tgataacact gcggccaact   4440
tacttctgac aacgatcgga ggaccgaagg agctaaccgc tttttttgcac aacatggggg   4500
atcatgtaac tcgccttgat cgttgggaac cggagctgaa tgaagccata ccaaacgacg   4560
agcgtgacac cacgatgcct gtagcaatgg caacaacgtt gcgcaaacta ttaactggcg   4620
aactacttac tctagcttcc cggcaacaat taatagactg gatggaggcg gataaagttg   4680
caggaccact tctgcgctcg gcccttccgg ctggctggtt tattgctgat aaatctggag   4740
ccggtgagcg tgggtctcgc ggtatcattg cagcactggg gccagatggt aagccctccc   4800
gtatcgtagt tatctacacg acggggagtc aggcaactat ggatgaacga aatagacaga   4860
tcgctgagat aggtgcctca ctgattaagc attggtaact gtcagactcg cgacactgca   4920
ttaatgaatc ggccaacgcg cggggagagg cggtttgcgt attgggcgct cttccgcttc   4980
ctcgctcact gactcgctgc gctcggtcgt tcggctgcgg cgagcggtat cagctcactc   5040
aaaggcggta atacggttat ccacagaatc aggggataac gcaggaaaga acatgtgagc   5100
aaaaggccag caaaaggcca ggaaccgtaa aaaggccgcg ttgctggcgt ttttccatag   5160
gctccgcccc cctgacgagc atcacaaaaa tcgacgctca agtcagaggt ggcgaaaccc   5220
gacaggacta taaagatacc aggcgtttcc ccctggaagc tccctcgtgc gctctcctgt   5280
tccgaccctg ccgcttaccg gatacctgtc cgcctttctc ccttcgggaa gcgtggcgct   5340
ttctcatagc tcacgctgta ggtatctcag ttcggtgtag gtcgttcgct ccaagctggg   5400
ctgtgtgcac gaaccccccg ttcagcccga ccgctgcgcc ttatccggta actatcgtct   5460
tgagtccaac ccggtaagac acgacttatc gccactggca gcagccactg gtaacaggat   5520
tagcagagcg aggtatgtag gcggtgctac agagttcttg aagtggtggc ctaactacgg   5580
ctacactaga aggacagtat ttggtatctg cgctctgctg aagccagtta ccttcggaaa   5640
aagagttggt agctcttgat ccggcaaaca aaccaccgct ggtagcggtg gtttttttgt   5700
ttgcaagcag cagattacgc gcagaaaaaa aggatctcaa gaagatcctt tgatcttttc   5760
tacggggtct gacgctcagt ggaacgaaaa ctca                               5794
```

<210> 3
<211> 14119
<212> DNA
<213> Artificial

<220>
<223> Eukaryotic expression vector

<400> 3

cgttgtaaaa cgacggccag tgaattgtaa tacgactcac tatagggcga attgggtacc 60
gggccccccc tcgaagttta aacatttaaa tctagaagct tcaatgtttt tagcaccctc 120
tgtgtggagg aaaataatgc agattattct aattagtgta atatctaacc acattaaaat 180
atattacata gtaaactaca ctccataatt ttataaattt gactccccag ggtaataaac 240
tagtctctag tctgctcacc ttcaactgta caataaagtc ttggttcttt tgaaatagac 300

ctcaaatgag acacctaaaa ttcaaagtgt ctttacattt aaagacacct acaggaaagc 360
aggtaaaaga gccaggttaa aaacaaattc taaaaccact tagctgcagt taaacatata 420
gtaaagatgc actaaagttt cttactctgt aaatcccttc cacttcagga aatattccac 480
tttcccattc actacacgtc gatctagtac tttttccacg acaaattctt caggctctgc 540
ctcttcaact tttttactct ttccattctg tttttttccc atttttgct aaaataaaac 600
aaaagagaaa ttaagaaata ttcctcttga attttgagca cattttcaag gctcaattgc 660
ttatattatt atcacattcg acataaattt ttacttctat atcccagggc agacaccttc 720
tggaaagatt aaaagtcaac agacaataaa ataaaagaat gctttatctt gttcatttag 780
ttcaaactta caacccacca ccaaaataat acaataaaaa aacactatct ggaaacagtt 840
atttttttcc agtctttttt tttgagacag ggtctcacac tcttgtcgcc caggctggag 900
tgcagtggcg tgatctcagc tcactgcaac ctccgcctcc ccaggttcaa gcagttctca 960
tgcctcagcc tccagagtag ctgggattat aggcggatgc caccatgccg ggctaatttt 1020
ttttgtgttt ttattagaaa cagggtttca ccatgttgac caggctggtc tcaaactcct 1080
gacctgaagt gattcaccag cctgggcctc ccaaagtgct ggcattacag gcgtgagcca 1140
ctgcgcccgg ccctgtagtc ttaaaagacc aagtttacta attttcactc attttaacaa 1200
cactgcaaca aacaactatg caggaagtac ctaaagggtg atccagagaa gcaagtagta 1260
gtgacaggtc ttaggtgaac ctatgacaga ccttgtatcc acccccagat ggtaaaagcc 1320
ccagcccccct tctcaattca aatattaatg tcaaaagcat caatgataca gagaaaagat 1380
aaatgcagaa tgaaaacatg gttcaaaatc ctgataccaa ctgcagggtc aactatagag 1440
accactagga ggttcaatta aaggacaaga ttatttttcc ataatctctg tagataatat 1500
ttcctaccac ttagaacaaa actataaagc tatcacttca agagaccaac attacaaatt 1560
tatttttaatt ccctaaggtg aaaaaaatcc ttccttcctg gtttctcaag agaaagtcta 1620
tactggtaac caaattcact ttaaacaggc attttctttg gtatgacact atttaagaga 1680
agcaggaaac caacgtgaac cagctctttc caatggctca agatttccta tgagaggact 1740
aaaaatgggg aaaattttta tgagaggatt aaaaatgggg gaaaaaaaac cctgaaatgg 1800
ttaatcagaa gatcctatgg gctgagaagg aatccatctt aacatttcat cttaaagcaa 1860
atgctattgc cggggggcagt ggctcatgcc tgtaatccca gcactttggg aggccgaggt 1920
gggcagatca tctgaggtca ggagtttgag accagcctga ccaacatgga gaaaccccgt 1980
ttctactaaa aatacaaaat tagccaggca tagtggtgca tgcctgtaat cccagctact 2040
tgggaggctg aggcaggaga actgcttgaa cccaggaggc ttaagttgcg gtgagccaag 2100
atcacgccat tgcactctag cctggacaac aagagaaaaa ctctgtctca aaaaaacaca 2160
aaaacaaaaa acccaaatac tatttaaaaa agataaacct taattgctca atcattaaag 2220
ccatcccaca agtaaagcag caagcagaaa aaagttaaga acacctcaag gctacagaag 2280
gacatttcaa gctatgcagg catatgaagt gtgcagacag atatgtaaga aaggcctcaa 2340

gactgcaaaa gggcatttca agctatgcaa gcatataggt aacacataca cacacacaaa 2400
ataaaatccc ctgaaataca aaaacatgca gcaaacacct gacgtttttg gataccattt 2460
ctaagtcagg tgttatgatt ctcattagtc aagatacttg agtactgggc ccaaacagct 2520
ttctgccact gtacagtaca agaaggtagg aataatggtg ggaggagcaa agacaaactg 2580
taatagacag aagtgtatca gatacctata ctacatgaaa aacaaaacag ctactgccac 2640
aaagggagaa ggctaacaaa ataaagtcaa caataaatac agaaaatgaa aaggatacac 2700
actaaggttt acaaaaaaaa aaaggcagac aaaatgccat acagtattca ttcactacta 2760
tggcattcat aagctagttt caaatgctca ctattttctt ttatagtata tatttgcctt 2820
aacccagcac tttttccaa aagtggatga gtcaaaataa atttcccatt atttaagtga 2880
aattaacagc acacatatct cacaacacta atgaattttt aaaatggaaa gttaagaact 2940
tttaaagtgg ccaacctgtg atccttcaca aaataaacta aatacaataa cagaccccaa 3000
aggctatcaa ttgcgtgcaa aaacaacttc tgttttccag ggtaaacaga atctaatgca 3060
gaatctaatg cagggtaaac agacttaatg cagaatctaa tgatggcaca aattaaaaat 3120
cactaacgtg ccctttttag tgtgaaaccc agagagagca catacaagcc aaaaacaaat 3180
gctttatttt acctaggaga cattaacatt cacctttacg tgtttaagat taatgcaatg 3240
ttaaatattg tgaaaactgt aactttgaat ttcatgattt ttatgtgaat attccagggt 3300
ttaaaaaaac ttgtaacatg acatggctga ataagataaa aaaaaaatct agccttttct 3360
cccttctggc tcatatttgc gatttcgatc attttgttta aaaaacaaaa cactgcaatg 3420
aattaaactt aatattcttc tatgttttag agtaagttaa aacaagataa agtgaccaaa 3480
gtaatttgaa agattcaatg acttttgctc caacctaggt gcacaaggta ccttgttctt 3540
taaattgggc tttaatgaaa atacttctcc agaattctgg ggatttaaga aaaattatgc 3600
caaccaacaa gggctttacc attttatgta acatttttca acgctgcaaa aatgtgtgta 3660
tttctatttg aagataaaaa tcctcagcaa aatccacatt gcactgtcct tcaaagatta 3720
gccttctttg aactagttaa gacactatta agccaagcca gtatctccct gtaatgaatt 3780
cgtttttctc ttaattttcc cctgtaattt acactgggag agctgggaaa tatgtggatg 3840
taaatttctc agccacagag atgcaaagtt atactgtggg gaaaaaaaac ttgagttaaa 3900
tccttacata ttttaggttt tcattaactt accaatgtag ttttgttgga ggccattttt 3960
tttattgcag acttgaagag ctattactag aaaaatgcat gacagttaag gtaagtttgc 4020
atgacacaaa aaaggtaact aaatacaaat tctgtttgga ttccaacccc caagtagaga 4080
gcgcacactt tcaaacgtga atacaaatcc agagtagatc tgcgctccta cctacattgc 4140
ttatgatgta cttaagtacg tgtcctaacc atgtgagtct agaaagactt tactggggat 4200
cctggtacct aaaacagctt cacatggctt aaaatagggg accaatgtct tttccaatct 4260
aagtcccatt tataataaag tccatgttcc atttttaaag gacaatcctt tcggtttaaa 4320
accaggcacg attacccaaa caactcacaa cggtaaagca ctgtgaatct tctctgttct 4380

gcaatcccaa cttggtttct gctcagaaac cctccctctt tccaatcggt aattaaataa 4440
caaaaggaaa aaacttaaga tgcttcaacc ccgtttcgtg acactttgaa aaaagaatca 4500
cctcttgcaa acacccgctc ccgacccccg ccgctgaagc ccggcgtcca gaggcctaag 4560
cgcgggtgcc cgcccccacc cgggagcgcg ggcctcgtgg tcagcgcatc cgcggggaga 4620
aacaaaggcc gcggcacggg ggctcaaggg cactgcgcca caccgcacgc gcctaccccc 4680
gcgcggccac gttaactggc ggtcgccgca gcctcgggac agccggccgc gcgccgccag 4740
gctcgcggac gcgggaccac gcgccgccct ccgggaggcc caagtctcga cccagccccg 4800
cgtggcgctg ggggaggggg cgcctccgcc ggaacgcggg tgggggaggg gagggggaaa 4860
tgcgctttgt ctcgaaatgg ggcaaccgtc gccacagttc cctacccccct cgagggcaga 4920
gcagtccccc cactaactac cgggctggcc gcgcgccagg ccagccgcga ggccaccgcc 4980
cgaccctcca ctccttcccg cagctcccgg cgcggggtcc ggcgagaagg ggaggggagg 5040
ggagcggaga accgggcccc cgggacgcgt gtggcatctg aagcaccacc agcgagcgag 5100
agctagagag aaggaaagcc accgacttca ccgcctccga gctgctccgg gtcgcgggtc 5160
tgcagcgtct ccggccctcc gcgcctacag ctcaagccac atccgaaggg ggagggagcc 5220
gggagctgcg cgcggggccg ccgggggggag gggtggcacc gcccacgccg ggcggccacg 5280
aagggcgggg cagcgggcgc gcgcgcggcg gggggagggg ccggcgccgc gcccgctggg 5340
aattggggcc ctagggggag ggcggaggcg ccgacgaccg cggcacttac cgttcgcggc 5400
gtggcgcccg gtggtcccca aggggaggga agggggaggc ggggcgagga cagtgaccgg 5460
agtctcctca gcggtggctt ttctgcttgg cagcctcagc ggctggcgcc aaaaccggac 5520
tccgcccact tcctcgcccg ccggtgcgag ggtgtggaat cctccagacg ctgggggagg 5580
gggagttggg agcttaaaaa ctagtacccc tttgggacca ctttcagcag cgaactctcc 5640
tgtacaccag gggtcagttc cacagacgcg ggccaggggt gggtcattgc ggcgtgaaca 5700
ataatttgac tagaagttga ttcgggtgtt tccggaaggg gccgagtcaa tccgccgagt 5760
tggggcacgg aaaacaaaaa gggaaggcta ctaagatttt tctggcgggg gttatcattg 5820
gcgtaactgc agggaccacc tcccgggttg agggggctgg atctccaggc tgcggattaa 5880
gcccctcccg tcggcgttaa tttcaaactg cgcgacgttt ctcacctgcc ttcgccaagg 5940
caggggccgg gaccctattc caagaggtag taactagcag gactctagcc ttccgcaatt 6000
cattgagcgc atttacggaa gtaacgtcgg gtactgtctc tggccgcaag ggtgggagga 6060
gtacgcattt ggcgtaaggt ggggcgtaga gccttcccgc cattggcggc ggatagggcg 6120
tttacgcgac ggcctgacgt agcggaagac gcgttagtgg gggggaaggt tctagaaaag 6180
cggcggcagc ggctctagcg gcagtagcag cagcgccggg tcccgtgcgg aggtgctcct 6240
cgcagagttg tttctcgagc agcggcagtt ctcactacag cgccaggacg agtccggttc 6300
gtgttcgtcc gcggagatct ctctcatctc gctcggctgc gggaaatcgg gctgaagcga 6360
ctgagtccgc gatggaggta acgggtttga aatcaatgag ttattgaaaa gggcatggcg 6420

aggccgttgg cgcctcagtg gaagtcggcc agccgcctcc gtgggagaga ggcaggaaat 6480
cggaccaatt cagtagcagt ggggcttaag gtttatgaac ggggtcttga gcggaggcct 6540
gagcgtacaa acagcttccc caccctcagc ctcccggcgc catttccctt cactgggggt 6600
gggggatggg gagctttcac atggcggacg ctgccccgct ggggtgaaag tggggcgcgg 6660
aggcgggaat tcttattccc tttctaaagc acgctgcttc gggggccacg gcgtctcctc 6720
ggcgagcgtt tcggcgggca gcaggtcctc gtgagcgagg ctgcggagct tcccctcccc 6780
ctctctcccg ggaaccgatt tggcggccgc cattttcatg gctcgccttc ctctcagcgt 6840
tttccttata actcttttat tttcttagtg tgctttctct atcaagaagt agaagtggtt 6900
aactattttt tttttcttct cgggctgttt tcatatcgtt tcgaggtgga tttggagtgt 6960
tttgtgagct tggatcttta gagtcctgcg cacctcatta aaggcgctca gccttcccct 7020
cgatgaaatg gcgccattgc gttcggaagc cacaccgaag agcggggagg gggggtgctc 7080
cgggtttgcg ggcccggttt cagagaagat atcaccaccc agggcgtcgg gccgggttca 7140
atgcgagccg taggacaaag aaaccatttt atgtttttcc tgtctttttt ttcctttgag 7200
taacggtttt atctgggtct gcagtcagta aaacgacaga tgaaccgcgg caaaataaac 7260
ataaattgga agccatcggc cacgaggggc agggacgaag gtggttttct gggcgggggga 7320
gggatattcg cgtcagaatc ctttactgtt cttaaggatt ccgtttaagt tgtagagctg 7380
actcatttta agtaatgttg ttactgagaa gtttaaccct tacgggacag atccatggac 7440
ctttatagat gattacgagg aaagtgaaat aacgattttg tccttagtta tacttcgatt 7500
aaaacatggc ttcagaggct ccttcctgta atgcgtatgg attgatgtgc aaaactgttt 7560
tgggcctggg ccgctctgta tttgaacttt gttacttttc tcattttgtt tgcaatcttg 7620
gttgaacatt acattgataa gcataaggtc tcaagcgaag gggtctacc tggttatttt 7680
tctttgaccc taagcacgtt tataaaataa cattgtttaa aatcgatagt ggacatcggg 7740
taagtttgga taaattgtga ggtaagtaat gagtttttgc tttttgttag tgatttgtaa 7800
aacttgttat aaatgtacat tatccgtaat ttcagtttag agataaccta tgtgctgacg 7860
acaattaaga ataaaaacta gctgaaaaaa tgaaaataac tatcgtgaca agtaaccatt 7920
tcaaaagact gctttgtgtc tcataggagc tagtttgatc atttcagtta atttttttctt 7980
taatttttac gagtcatgaa aactacagga aaaaaaatct gaactgggtt ttaccactac 8040
tttttaggag ttgggagcat gcgaatggag ggagagctcc gtagaactgg gatgagagca 8100
gcaattaatg ctgcttgcta ggaacaaaaa ataattgatt gaaaattacg tgtgactttt 8160
tagtttgcat tatgcgtttg tagcagttgg tcctggatat cactttctct cgtttgaggt 8220
tttttaacct agttaacttt taagacaggt ttccttaaca ttcataagtg cccagaatac 8280
agctgtgtag tacagcatat aaagatttca gctctgaggt tttcctatt gacttggaaa 8340
attgttttgt gcctgtcgct tgccacatgg ccaatcaagt aagcttatcg ataccggtgg 8400
agctcaagct tcgaattctg cagtcgacgg taccgcgggc ccgggatcta tgttacttgg 8460

cagaggccgc atggaaagtc cctggacgtg ggacatctga ttaatacgtg aggaggtcag 8520
ccatgttctt tttggcaaag gactacggtc attggacgtt tgattggcat gggatagggt 8580
cagccagagt taacagtgtt cttttggcaa agggatacgt ggaaagtccc gggccattta 8640
cagtaaactg atacggggac aaagcacagc catatttagt catgtattgc ttggcagagg 8700
gtctatggaa agtccctgga cgtgggacgt ctgattaata tgaaagaagg tcagccagag 8760
gtagctgtgt cctttttggc aaagggatac ggttatggga cgtttgattg gactgggata 8820
gggtcagcca gagttaacag tgttcttttg gcaaaggaaa cgtggaaagt cccgggccat 8880
ttacagtaaa ctgatactgg gacaaagtac acccatattt agtcatgttc tttttggcaa 8940
agagcatctg gaaagtcccg ggcagcatta tagtcacttg gcagagggaa agggtcactc 9000
agagttaagt acatctttcc agggccaata ttccagtaaa ttacacttag ttttatgcaa 9060
atcagccaca aaggggattt tcccggtcaa ttatgacttt ttccttagtc atgcggtatc 9120
caattactgc caaattggca gtacatacta ggtgattcac tgacatttgg ccgtcctctg 9180
gaaagtccct ggaaaccgct caagtactgt atcatggtga ctttgcattt ttggagagca 9240
cgccccactc caccattggt ccacgtaccc tatgggggag tggtttatga gtatataagg 9300
ggctccggtt tagaagccgg gcagagcgga attgatccac cggtcgccac catggtgagc 9360
aagggcgagg agctgttcac cggggtggtg cccatcctgg tcgagctgga cggcgacgta 9420
aacggccaca agttcagcgt gtccggcgag ggcgagggcg atgccaccta cggcaagctg 9480
accctgaagt tcatctgcac caccggcaag ctgcccgtgc cctggcccac cctcgtgacc 9540
accctgacct acggcgtgca gtgcttcagc cgctaccccg accacatgaa gcagcacgac 9600
ttcttcaagt ccgccatgcc cgaaggctac gtccaggagc gcaccatctt cttcaaggac 9660
gacggcaact acaagacccg cgccgaggtg aagttcgagg gcgacaccct ggtgaaccgc 9720
atcgagctga agggcatcga cttcaaggag gacggcaaca tcctggggca caagctggag 9780
tacaactaca acagccacaa cgtctatatc atggccgaca agcagaagaa cggcatcaag 9840
gtgaacttca agatccgcca caacatcgag gacggcagcg tgcagctcgc cgaccactac 9900
cagcagaaca cccccatcgg cgacggcccc gtgctgctgc ccgacaacca ctacctgagc 9960
acccagtccg ccctgagcaa agaccccaac gagaagcgcg atcacatggt cctgctggag 10020
ttcgtgaccg ccgccgggat cactctcggc atggacgagc tgtacaagta aagcggccgc 10080
gactctagat cataatcagc cataccacat ttgtagaggt tttacttgct ttaaaaaacc 10140
tcccacacct ccccctgaac ctgaaacata aaatgaatgc aattgttgtt gttaacttgt 10200
ttattgcagc ttataatggt tacaaataaa gcaatagcat cacaaatttc acaaataaag 10260
catttttttc actgcattct agttgtggtt tgtccaaact catcaatgta tcttaactag 10320
agtcgacctg caggcatgca agcttaccgg tggcgcgcgc gccaattgtt aattaagatc 10380
tggcccaatg ggccgtacga attccttagg ctaccgggta ggggaggcgc ttttcccaag 10440
gcagtctgga gcatgcgctt tagcagcccc gctgggcact tggcgctaca caagtggcct 10500

ctggcctcgc acacattcca catccaccgg ccggtaggcg ccaaccggct ccgttctttg 10560
gtggcccctt cgcgccacct tctactcctc ccctagtcag gaagttcccc cccgccccgc 10620
agctcgcgtc gtgcaggacg tgacaaatgg aagtagcacg tctcactagt ctcgtgcaga 10680
tggacagcac cgctgagcaa tggaagcggg taggcctttg gggcagcggc caatagcagc 10740
tttgctcctt cgctttctgg gctcagaggc tgggaagggg tgggtccggg ggcgggctca 10800
ggggcgggct caggggcggg gcgggcgccc gaaggtcctc cggaggcccg gcattctgca 10860
cgcttcaaaa gcgcacgtct gccgcgctgt tctcctcttc ctcatctccg ggcctttcga 10920
ccagcttacc atgaccgagt acaagcccac ggtgcgcctc gccacccgcg acgacgtccc 10980
cagggccgta cgcaccctcg ccgccgcgtt cgccgactac cccgccacgc gccacaccgt 11040
cgatccggac cgccacatcg agcgggtcac cgagctgcaa gaactcttcc tcacgcgcgt 11100
cgggctcgac atcggcaagg tgtgggtcgc ggacgacggc gccgcggtgg cggtctggac 11160
cacgccggag agcgtcgaag cgggggcggt gttcgccgag atcggcccgc gcatggccga 11220
gttgagcggt tcccggctgg ccgcgcagca acagatggaa ggcctcctgg cgccgcaccg 11280
gcccaaggag cccgcgtggt tcctggccac cgtcggcgtc tcgcccgacc accagggcaa 11340
gggtctgggc agcgccgtcg tgctccccgg agtggaggcg gccgagcgcg ccggggtgcc 11400
cgccttcctg gagacctccg cgccccgcaa cctccccttc tacgagcggc tcggcttcac 11460
cgtcaccgcc gacgtcgagg tgcccgaagg accgcgcacc tggtgcatga cccgcaagcc 11520
cggtgcctga cgcccgcccc acgacccgca gcgcccgacc gaaaggagcg cacgacccca 11580
tgcatcgtag acgaaatgac cgaccaagcg acgcccaacc tgccatcacg agatttcgat 11640
tccaccgccg ccttctatga aaggttgggc ttcggaatcg ttttccggga cgccggctgg 11700
atgatcctcc agcgcgggga tctcatgctg gagttcttcg cccaccctag ggggaggcta 11760
actgaaacac ggaaggagac aataccggaa ggaacccgcg ctatgacggc aataaaaaga 11820
cagaataaaa cgcacggtgt tgggtcgttt gttcataaac gcggggttcg gtcccagggc 11880
tggcactctg tcgatacccc accgagaccc cattggggcc aatacgcccg cgtttcttcc 11940
ttttccccac cccacccccc aagttcgggt gaaggcccag ggctcgcagc caacgtcggg 12000
gcggcaggcc cccagctttt gttcccttta gtgagggtta atttcgagct tggcgtaatc 12060
atggtcatag ctgtttcctg tgtgaaattg ttatccgctc acaattccac acaacatacg 12120
agccggaagc ataaagtgta aagcctgggg tgcctaatga gtgagctaac tcacattaat 12180
tgcgttgcgc tcactgcccg ctttccagtc gggaaacctg tcgtgccagc atcgcgagca 12240
cttttcgggg aaatgtgcgc ggaaccccta tttgtttatt tttctaaata cattcaaata 12300
tgtatccgct catgagacaa taaccctgat aaatgcttca ataatattga aaaaggaaga 12360
gtatgagtat tcaacatttc cgtgtcgccc ttattccctt ttttgcggca ttttgccttc 12420
ctgtttttgc tcacccagaa acgctggtga aagtaaaaga tgctgaagat cagttgggtg 12480
cacgagtggg ttacatcgaa ctggatctca acagcggtaa gatccttgag agttttcgcc 12540

ccgaagaacg ttttccaatg atgagcactt ttaaagttct gctatgtggc gcggtattat 12600
cccgtattga cgccgggcaa gagcaactcg gtcgccgcat acactattct cagaatgact 12660
tggttgagta ctcaccagtc acagaaaagc atcttacgga tggcatgaca gtaagagaat 12720
tatgcagtgc tgccataacc atgagtgata acactgcggc caacttactt ctgacaacga 12780
tcggaggacc gaaggagcta accgcttttt tgcacaacat gggggatcat gtaactcgcc 12840
ttgatcgttg ggaaccggag ctgaatgaag ccataccaaa cgacgagcgt gacaccacga 12900
tgcctgtagc aatggcaaca acgttgcgca aactattaac tggcgaacta cttactctag 12960
cttcccggca acaattaata gactggatgg aggcggataa agttgcagga ccacttctgc 13020
gctcggccct tccggctggc tggtttattg ctgataaatc tggagccggt gagcgtgggt 13080
ctcgcggtat cattgcagca ctggggccag atggtaagcc ctcccgtatc gtagttatct 13140
acacgacggg gagtcaggca actatggatg aacgaaatag acagatcgct gagataggtg 13200
cctcactgat taagcattgg taactgtcag actcgcgaca ctgcattaat gaatcggcca 13260
acgcgcgggg agaggcggtt tgcgtattgg gcgctcttcc gcttcctcgc tcactgactc 13320
gctgcgctcg gtcgttcggc tgcggcgagc ggtatcagct cactcaaagg cggtaatacg 13380
gttatccaca gaatcagggg ataacgcagg aaagaacatg tgagcaaaag gccagcaaaa 13440
ggccaggaac cgtaaaaagg ccgcgttgct ggcgtttttc cataggctcc gcccccctga 13500
cgagcatcac aaaaatcgac gctcaagtca gaggtggcga aacccgacag gactataaag 13560
ataccaggcg tttccccctg gaagctccct cgtgcgctct cctgttccga ccctgccgct 13620
taccggatac ctgtccgcct ttctcccttc gggaagcgtg gcgctttctc atagctcacg 13680
ctgtaggtat ctcagttcgg tgtaggtcgt tcgctccaag ctgggctgtg tgcacgaacc 13740
ccccgttcag cccgaccgct gcgccttatc cggtaactat cgtcttgagt ccaacccggt 13800
aagacacgac ttatcgccac tggcagcagc cactggtaac aggattagca gagcgaggta 13860
tgtaggcggt gctacagagt tcttgaagtg gtggcctaac tacggctaca ctagaaggac 13920
agtatttggt atctgcgctc tgctgaagcc agttaccttc ggaaaaagag ttggtagctc 13980
ttgatccggc aaacaaacca ccgctggtag cggtggtttt tttgtttgca agcagcagat 14040
tacgcgcaga aaaaaaggat ctcaagaaga tcctttgatc ttttctacgg ggtctgacgc 14100
tcagtggaac gaaaactca 14119

<210> 4
<211> 7383
<212> DNA
<213> Artificial

<220>
<223> Eukaryotic expression vector

<400> 4

cgttgtaaaa cgacggccag tgaattgtaa tacgactcac tatagggcga attgggtacc 60
gggccccccc tcgaagttta aacatttcta gcgctaccgg actcagatcc cctccgcgcc 120

tacagctcaa gccacatccg aagggggagg gagccgggag ctgcgcgcgg ggccgccggg 180
gggaggggtg gcaccgccca cgccgggcgg ccacgaaggg cggggcagcg ggcgcgcgcg 240
cggcgggggg aggggccggc gccgcgcccg ctgggaattg gggccctagg gggagggcgg 300
aggcgccgac gaccgcggca cttaccgttc gcggcgtggc gcccggtggt ccccaagggg 360
agggaagggg gaggcggggc gaggacagtg accggagtct cctcagcggt ggcttttctg 420
cttggcagcc tcagcggctg gcgccaaaac cggactccgc ccacttcctc gcccgccggt 480
gcgagggtgt ggaatcctcc agacgctggg ggagggggag ttgggagctt aaaaactagt 540
acccctttgg gaccactttc agcagcgaac tctcctgtac accaggggtc agttccacag 600
acgcgggcca ggggtgggtc attgcggcgt gaacaataat ttgactagaa gttgattcgg 660
gtgtttccgg aaggggccga gtcaatccgc cgagttgggg cacggaaaac aaaaagggaa 720
ggctactaag atttttctgg cgggggttat cattggcgta actgcaggga ccacctcccg 780
ggttgagggg gctggatctc caggctgcgg attaagcccc tcccgtcggc gttaatttca 840
aactgcgcga cgtttctcac ctgccttcgc caaggcaggg gccgggaccc tattccaaga 900
ggtagtaact agcaggactc tagccttccg caattcattg agcgcattta cggaagtaac 960
gtcgggtact gtctctggcc gcaagggtgg gaggagtacg catttggcgt aaggtggggc 1020
gtagagcctt cccgccattg gcggcggata gggcgtttac gcgacggcct gacgtagcgg 1080
aagacgcgtt agtggggggg aaggttctag aaaagcggcg gcagcggctc tagcggcagt 1140
agcagcagcg ccgggtcccg tgcggaggtg ctcctcgcag agttgtttct cgagcagcgg 1200
cagttctcac tacagcgcca ggacgagtcc ggttcgtgtt cgtccgcgga gatctctctc 1260
atctcgctcg gctgcgggaa atcgggctga agcgactgag tccgcgatgg aggtaacggg 1320
tttgaaatca atgagttatt gaaaagggca tggcgaggcc gttggcgcct cagtggaagt 1380
cggccagccg cctccgtggg agagaggcag gaaatcggac caattcagta gcagtggggc 1440
ttaaggttta tgaacggggt cttgagcgga ggcctgagcg tacaaacagc ttccccaccc 1500
tcagcctccc ggcgccattt cccttcactg ggggtggggg atggggagct ttcacatggc 1560
ggacgctgcc ccgctggggt gaaagtgggg cgcggaggcg ggaattctta ttccctttct 1620
aaagcacgct gcttcggggg ccacggcgtc tcctcgggat ctcgagctca agcttcgaat 1680
tctgcagtcg acggtaccgc gggcccggga tctatgttac ttggcagagg ccgcatggaa 1740
agtccctgga cgtgggacat ctgattaata cgtgaggagg tcagccatgt tctttttggc 1800
aaaggactac ggtcattgga cgtttgattg gcatgggata gggtcagcca gagttaacag 1860
tgttcttttg gcaaagggat acgtggaaag tcccgggcca tttacagtaa actgatacgg 1920
ggacaaagca cagccatatt tagtcatgta ttgcttggca gagggtctat ggaaagtccc 1980
tggacgtggg acgtctgatt aatatgaaag aaggtcagcc agaggtagct gtgtcctttt 2040
tggcaaaggg atacggttat gggacgtttg attggactgg gatagggtca gccagagtta 2100
acagtgttct tttggcaaag gaaacgtgga aagtcccggg ccatttacag taaactgata 2160

ctgggacaaa gtacacccat atttagtcat gttctttttg gcaaagagca tctggaaagt 2220
cccgggcagc attatagtca cttggcagag ggaaagggtc actcagagtt aagtacatct 2280
ttccagggcc aatattccag taaattacac ttagttttat gcaaatcagc cacaaagggg 2340
attttcccgg tcaattatga ctttttcctt agtcatgcgg tatccaatta ctgccaaatt 2400
ggcagtacat actaggtgat tcactgacat ttggccgtcc tctggaaagt ccctggaaac 2460
cgctcaagta ctgtatcatg gtgactttgc atttttggag agcacgcccc actccaccat 2520
tggtccacgt accctatggg ggagtggttt atgagtatat aaggggctcc ggtttagaag 2580
ccgggcagag cggaattgat ccaccggtcg ccaccatggt gagcaagggc gaggagctgt 2640
tcaccggggt ggtgcccatc ctggtcgagc tggacggcga cgtaaacggc cacaagttca 2700
gcgtgtccgg cgagggcgag ggcgatgcca cctacggcaa gctgaccctg aagttcatct 2760
gcaccaccgg caagctgccc gtgccctggc ccaccctcgt gaccaccctg acctacggcg 2820
tgcagtgctt cagccgctac cccgaccaca tgaagcagca cgacttcttc aagtccgcca 2880
tgcccgaagg ctacgtccag gagcgcacca tcttcttcaa ggacgacggc aactacaaga 2940
cccgcgccga ggtgaagttc gagggcgaca ccctggtgaa ccgcatcgag ctgaagggca 3000
tcgacttcaa ggaggacggc aacatcctgg ggcacaagct ggagtacaac tacaacagcc 3060
acaacgtcta tatcatggcc gacaagcaga agaacggcat caaggtgaac ttcaagatcc 3120
gccacaacat cgaggacggc agcgtgcagc tcgccgacca ctaccagcag aacaccccca 3180
tcggcgacgg ccccgtgctg ctgcccgaca accactacct gagcacccag tccgccctga 3240
gcaaagaccc caacgagaag cgcgatcaca tggtcctgct ggagttcgtg accgccgccg 3300
ggatcactct cggcatggac gagctgtaca agtaaagcgg ccgcgactct agatcataat 3360
cagccatacc acatttgtag aggttttact tgctttaaaa aacctcccac acctccccct 3420
gaacctgaaa cataaaatga atgcaattgt tgttgttaac ttgtttattg cagcttataa 3480
tggttacaaa taaagcaata gcatcacaaa tttcacaaat aaagcatttt tttcactgca 3540
ttctagttgt ggtttgtcca aactcatcaa tgtatcttaa ctagagtcga cctgcaggca 3600
tgcaagctta ccggtggcgc gcgcgccaat tgttaattaa gatctggccc aatgggccgt 3660
acgaattcct taggctaccg ggtaggggag gcgcttttcc caaggcagtc tggagcatgc 3720
gctttagcag ccccgctggg cacttggcgc tacacaagtg gcctctggcc tcgcacacat 3780
tccacatcca ccggccggta ggcgccaacc ggctccgttc tttggtggcc ccttcgcgcc 3840
accttctact cctcccctag tcaggaagtt cccccccgcc ccgcagctcg cgtcgtgcag 3900
gacgtgacaa atggaagtag cacgtctcac tagtctcgtg cagatggaca gcaccgctga 3960
gcaatggaag cgggtaggcc tttggggcag cggccaatag cagctttgct ccttcgcttt 4020
ctgggctcag aggctgggaa ggggtgggtc cgggggcggg ctcaggggcg ggctcagggg 4080
cggggcgggc gcccgaaggt cctccggagg cccggcattc tgcacgcttc aaaagcgcac 4140
gtctgccgcg ctgttctcct cttcctcatc tccgggcctt tcgaccagct taccatgacc 4200

gagtacaagc ccacggtgcg cctcgccacc cgcgacgacg tccccagggc cgtacgcacc 4260
ctcgccgccg cgttcgccga ctaccccgcc acgcgccaca ccgtcgatcc ggaccgccac 4320
atcgagcggg tcaccgagct gcaagaactc ttcctcacgc gcgtcgggct cgacatcggc 4380
aaggtgtggg tcgcggacga cggcgccgcg gtggcggtct ggaccacgcc ggagagcgtc 4440
gaagcggggg cggtgttcgc cgagatcggc ccgcgcatgg ccgagttgag cggttcccgg 4500
ctggccgcgc agcaacagat ggaaggcctc ctggcgccgc accggcccaa ggagcccgcg 4560
tggttcctgg ccaccgtcgg cgtctcgccc gaccaccagg gcaagggtct gggcagcgcc 4620
gtcgtgctcc ccggagtgga ggcggccgag cgcgccgggg tgcccgcctt cctggagacc 4680
tccgcgcccc gcaacctccc cttctacgag cggctcggct tcaccgtcac cgccgacgtc 4740
gaggtgcccg aaggaccgcg cacctggtgc atgacccgca agcccggtgc ctgacgcccg 4800
ccccacgacc cgcagcgccc gaccgaaagg agcgcacgac cccatgcatc gtagacgaaa 4860
tgaccgacca agcgacgccc aacctgccat cacgagattt cgattccacc gccgccttct 4920
atgaaaggtt gggcttcgga atcgttttcc gggacgccgg ctggatgatc ctccagcgcg 4980
gggatctcat gctggagttc ttcgcccacc ctaggggggag gctaactgaa acacggaagg 5040
agacaatacc ggaaggaacc cgcgctatga cggcaataaa aagacagaat aaaacgcacg 5100
gtgttgggtc gtttgttcat aaacgcgggg ttcggtccca gggctggcac tctgtcgata 5160
ccccaccgag accccattgg ggccaatacg cccgcgtttc ttccttttcc ccaccccacc 5220
ccccaagttc gggtgaaggc ccagggctcg cagccaacgt cggggcggca ggcccccagc 5280
ttttgttccc tttagtgagg gttaatttcg agcttggcgt aatcatggtc atagctgttt 5340
cctgtgtgaa attgttatcc gctcacaatt ccacacaaca tacgagccgg aagcataaag 5400
tgtaaagcct ggggtgccta atgagtgagc taactcacat taattgcgtt gcgctcactg 5460
cccgctttcc agtcgggaaa cctgtcgtgc cagcatcgcg agcacttttc ggggaaatgt 5520
gcgcggaacc cctatttgtt tatttttcta aatacattca aatatgtatc cgctcatgag 5580
acaataaccc tgataaatgc ttcaataata ttgaaaaagg aagagtatga gtattcaaca 5640
tttccgtgtc gcccttattc ccttttttgc ggcattttgc cttcctgttt ttgctcaccc 5700
agaaacgctg gtgaaagtaa aagatgctga agatcagttg ggtgcacgag tgggttacat 5760
cgaactggat ctcaacagcg gtaagatcct tgagagtttt cgccccgaag aacgttttcc 5820
aatgatgagc acttttaaag ttctgctatg tggcgcggta ttatcccgta ttgacgccgg 5880
gcaagagcaa ctcggtcgcc gcatacacta ttctcagaat gacttggttg agtactcacc 5940
agtcacagaa aagcatctta cggatggcat gacagtaaga gaattatgca gtgctgccat 6000
aaccatgagt gataacactg cggccaactt acttctgaca acgatcggag gaccgaagga 6060
gctaaccgct tttttgcaca acatggggga tcatgtaact cgccttgatc gttgggaacc 6120
ggagctgaat gaagccatac caaacgacga gcgtgacacc acgatgcctg tagcaatggc 6180
aacaacgttg cgcaaactat taactggcga actacttact ctagcttccc ggcaacaatt 6240

```
aatagactgg atggaggcgg ataaagttgc aggaccactt ctgcgctcgg cccttccggc    6300
tggctggttt attgctgata aatctggagc cggtgagcgt gggtctcgcg gtatcattgc    6360
agcactgggg ccagatggta agccctcccg tatcgtagtt atctacacga cggggagtca    6420
ggcaactatg gatgaacgaa atagacagat cgctgagata ggtgcctcac tgattaagca    6480
ttggtaactg tcagactcgc gacactgcat taatgaatcg gccaacgcgc ggggagaggc    6540
ggtttgcgta ttgggcgctc ttccgcttcc tcgctcactg actcgctgcg ctcggtcgtt    6600
cggctgcggc gagcggtatc agctcactca aaggcggtaa tacggttatc cacagaatca    6660
ggggataacg caggaaagaa catgtgagca aaaggccagc aaaaggccag gaaccgtaaa    6720
aaggccgcgt tgctggcgtt tttccatagg ctccgccccc ctgacgagca tcacaaaaat    6780
cgacgctcaa gtcagaggtg gcgaaacccg acaggactat aaagatacca ggcgtttccc    6840
cctggaagct ccctcgtgcg ctctcctgtt ccgaccctgc cgcttaccgg atacctgtcc    6900
gcctttctcc cttcgggaag cgtggcgctt tctcatagct cacgctgtag gtatctcagt    6960
tcggtgtagg tcgttcgctc caagctgggc tgtgtgcacg aaccccccgt tcagcccgac    7020
cgctgcgcct tatccggtaa ctatcgtctt gagtccaacc cggtaagaca cgacttatcg    7080
ccactggcag cagccactgg taacaggatt agcagagcga ggtatgtagg cggtgctaca    7140
gagttcttga agtggtggcc taactacggc tacactagaa ggacagtatt tggtatctgc    7200
gctctgctga agccagttac cttcggaaaa agagttggta gctcttgatc cggcaaacaa    7260
accaccgctg gtagcggtgg tttttttgtt tgcaagcagc agattacgcg cagaaaaaaa    7320
ggatctcaag aagatccttt gatcttttct acggggtctg acgctcagtg gaacgaaaac    7380
tca                                                                  7383
```

<210> 5
<211> 33
<212> DNA
<213> Artificial

<220>
<223> oligonucleotide

<400> 5
tgtcgcgagt ctgacagtta ccaatgctta atc 33

<210> 6
<211> 33
<212> DNA
<213> Artificial

<220>
<223> oligonucleotide

<400> 6
catcgcgagc acttttcggg gaaatgtgtg cgc 33

<210> 7

<211> 33
<212> DNA
<213> Artificial

<220>
<223> oligonucleotide

<400> 7
tcgaagttta aacatttaaa tctagaagct tat 33

<210> 8
<211> 38
<212> DNA
<213> Artificial

<220>
<223> oligonucleotide

<400> 8
ccggtatcga taagcttcta gatttaaatg tttaaact 38

<210> 9
<211> 38
<212> DNA
<213> Artificial

<220>
<223> oligonucleotide

<400> 9
cgataccggt ggcgcgccaa ttgttaatta agatctgg 38

<210> 10
<211> 38
<212> DNA
<213> Artificial

<220>
<223> oligonucleotide

<400> 10
cccattgggc cagatcttaa ttaacaattg gcgcgcca 38

<210> 11
<211> 33
<212> DNA
<213> Artificial

<220>
<223> Oligonucleotide

<400> 11
cccaatgggc cgtacgaatt ccttaggctc gag 33

<210> 12
<211> 28
<212> DNA
<213> Artificial

<220>
<223> oligonucleotide

<400> 12
ggccctcgag cctaaggaat tcgtacgg 28